# NEW EUROPEAN PATENT SPECIFICATION

(11) **EP 1 214 097 B3**
(45) Date of publication of this specification: **20.07.2011**
(45) Mention of the grant of the patent: 15.07.2009
(21) Application number: 00965119.1
(22) Date of filing: 18.09.2000
(51) Int. Cl.: A61K 48/00, A61K 39/12, C07H 21/04

(54) **NUCLEIC ACIDS ENCODING POLYEPITOPE POLYPEPTIDES**
FÜR POLYEPITOP-POLYPEPTIDE KODIERENDE NUKLEINSÄUREN
ACIDES NUCLEIQUES CODANT POUR DES POLYPEPTIDES DE POLYEPITOPES

(30) Priority: 16.09.1999 US 398534; 16.09.1999 US 154665 P; 09.12.1999 US 458173; 09.12.1999 US 169846 P
(43) Date of publication of application: 19.06.2002
(60) Divisional application: 09008339.5
(73) Proprietor: Eisai Inc., Woodcliff Lake, NJ 07677 (US)
(72) Inventor: HEDLEY, Mary, Lynne, Lexington, MA 02173 (US); URBAN, Robert, C., Lexington, MA 02173 (US); CHICZ, Roman, M., Belmont, MA 02478 (US)
(74) Representative: Dzieglewska, Hanna Eva
(86) International application number: PCT/US2000/025559
(87) International publication number: WO 2001/019408

(56) References cited:
- EP-A2- 0 451 550
- WO-A-99/18995
- WO-A1-92/05248
- WO-A1-96/19496
- US-A- 5 629 161
- US-A- 5 679 509
- US-A- 5 719 054
- US-A- 5 753 233
- BOURSNELL M E G ET AL: "Construction and characterisation of a recombinant vaccinia virus expressing human papillomavirus proteins for immunotherapy of cervical cancer" VACCINE, BUTTERWORTH SCIENTIFIC. GUILDFORD, GB, vol. 14, no. 16, November 1996 (1996-11), pages 1485-1494, XP004063302 ISSN: 0264-410X
- MURAKAMI M ET AL: "HUMAN PAPILLOMAVIRUS VACCINES FOR CERVICAL CANCER" JOURNAL OF IMMUNOTHERAPY, RAVEN PRESS, NEW YORK, US, vol. 22, no. 3, May 1999 (1999-05), pages 212-218, XP008006675 ISSN: 1524-9557

## Description

### Field of the Invention

This invention relates generally to vaccines, and in particular to nucleic acid vaccines.

### Background of the Invention

Papilloma viruses are non-enveloped DNA viruses with a double stranded circular genome of approximately 9,000 base pairs. Over 75 types of HPV have been typed at the DNA level, and these can be broadly grouped into families on the basis of their tissue tropism.

Histologic, molecular, and epidemiologic evidence have implicated some HPV strains in cervical dysplasia and cervical cancer. Many studies support the view that most moderate and severe cervical intraepithelial neoplasias (CIN) contain HPV DNA which is exclusively detected in the histologically abnormal epithelium of these lesions. Persistent infection with HPV is believed to be the predominant risk factor for development of cervical carcinoma. HPV DNA is readily found in episomal form within cells exhibiting a cytopathic effect, while the HPV DNA is found integrated within the chromosomes of cells associated with most high grade precancerous lesions and cancer. Approximately 23 HPV types are commonly found in anogenital screening programs, but only 10-15 are associated with progressive disease. Types 16 and 18 are commonly found in association with cervical dysplasia and cervical cancer.

Papilloma viruses contain nine open reading frames. HPV genes with transforming properties have been mapped to open reading frames E6 and E7. Substantial biochemical work has demonstrated that the HPV E6 protein inactivates the protein p53, whereas the E7 protein interferes with retinoblastoma (Rb) protein function. Since p53 and Rb are tumor-suppressor proteins that function as cell division inhibitors, their inactivation by E6 and E7 leads the cell to enter into S phase of the cell cycle. Expression of E6 and E7 is sufficient to immortalize some primary cell lines, and blocking E6 or E7 function has been shown to reverse the transformed state.

Boursnell *et al.* reports a recombinant virus vector encoding part or all of the HPV 16 E6, E7 and HPV 18 E6, E7 proteins (US 5719054, column 3, lines 2-35).

### Summary of the Invention

The invention is based in part on the discovery that polypeptide segments, each of which contains one or more antibody-binding or class I or class II MHC-binding epitopes, can be linked in tandem to form polyepitope polypeptides which are proteolytically processed in cells to release the epitopes. These hybrid polypeptides are useful for stimulating an immune response, particularly when expressed from a nucleic acid within an antigen-presenting cell (APC).

The invention features a nucleic acid encoding a hybrid polypeptide the sequence of which comprises a signal sequence and
(i) the following segments of human papilloma virus (HPV) strain 16 E6:
   AMFQDPQERPRKLPQLCTEL (SEQ ID NO:64),
   LLRREVYDFAFRDLCIVYRDGNPY (SEQ ID NO:65), and
   KISEYRHYCYSLYGTTLEQQYNK (SEQ ID NO:66);
(ii) the following segments of HPV strain 16 E7:
   TLHEYMLDLQPETTDLYSY (SEQ ID NO:67),
   QAEPDRAHYNTVTF (SEQ ID NO:68), and
   LLMGTLGIVCPICSQKP (SEQ ID NO:69);
(iii) the following segments of HPV strain 18 E6:
   RRPYKLPDLCTELNTSLQDIETTCVYCKTVLELTEVFEFAFK (SEQ ID NO: 152), and
   SVYGDTLEKLTNTGLYNLLIRCLRCQK (SEQ ID NO: 153), and
(iv) the following segments of HPV strain 18 E7:
   KATLQDIVLHLEPQNEIPV (SEQ ID NO:154),
   HTMLCMCCKCEARI (SEQ ID NO:155), and
   AFQQLFLNTLSFVCPWC (SEQ ID NO: 156), providedthatthe hybrid polypeptide does not comprise a sequence identical to the sequence of either full length, intact E6 or full length, intact E7 protein from HPV strain 16 or 18.

As used herein, a "segment" is an amino acid sequence which (a) corresponds to the sequence of a portion (i.e., fragment less than all) of a naturally occurring protein, and (b) contains one or more epitopes. For clarity, the term "segment" is used herein to denote a part of the hybrid polypeptide, while the term "portion" is used to denote the corresponding part of the naturally occurring protein. By "epitope" is meant a peptide which binds to the binding groove of an MHC class I or class II molecule or to the antigen-binding region of an antibody. A methionine codon is preferably included at the 5' end of this or Any other coding sequence of the invention, to facilitate translation. In addition, the hybrid polypeptide encodes a targeting signal, as described in more detail below.

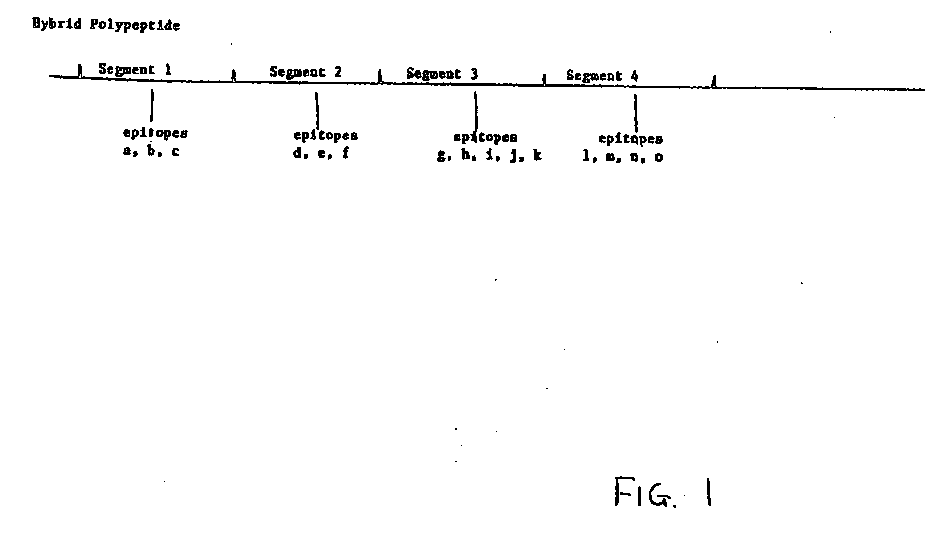

The relationship between segments and epitopes in the hybrid polypeptides is shown schematically in Fig. 1. The Illustrated hybrid polypeptide is composed of segments 1,2,3, and 4, each of which separately corresponds to a portion of a naturally occurring protein. Segment 1 can be proteolytically processed within a cell to generate epitopes a, b, and c. Similarly, segment 2 can be proteolytically processed to generate epitopes d, e, and f; segment 3 can be processed to generate epitopes g, h, i,j, and k; and segment 4, upon processing, yields epitopes 1, m, n and o. Adjacent segments can be contiguous, or can be separated by a spacer amino acid or spacer peptide.

The polypeptide may optionally include additional segments, e.g., it can include at least 4, 5, 10, 15, 20, 25, 30, 40, 50, 60, 75, 90, oreven 100 or more segments, each being a portion of a naturally-occurring protein of a pathogenic agent and/or of a naturally occurring tumor antigen which can be the same or different from the protein(s) from which the other segments are derived. Each of these segments can be at least 11 amino acids in length, and each contains at least one epitope (preferably two or more) different from the epitopes of the other segments. At least one preferably at least two or three) of the segments in the hybrid polypeptide may contain, e.g., 3, 4, 5, 6, 7, or even 10 or more epitopes, particularly class I or class II. MHC-binding epitopes. Two, three, or more of the segments can be contiguous in the hybrid polypeptide: i.e., they are joined end-to-end, with no spacer between them. Alternatively, any two adjacent segments can be linked by a spacer amino acid or spacer peptide.

When the hybrid polypeptide is introduced into a cell, it is proteolytically processed into at least some of its constituent epitopes. At least some of the epitopes generated from the segments in the polypeptide can bind to MHC class I or MHC class II molecules present in the cell, though some of the epitopes may be specific for MHC class I or class II molecules present only on other cells. The epitopes may alternatively be B cell epitopes which elicit antibody-mediated immune responses upon binding to antibody receptors on the surface of a B cell.

A given segment within the hybrid polypeptide need not be any specified length, so long as it is sufficiently long to generate at least one epitope, e.g., 2, 3, 4, 5, or more epitopes, and is at least 11 amino acids in length. For example, a given segment can have a length of at least 12 amino acids, e.g., at least 13, 14, 15, 20, 25, 30, 40, or 50 amino acids. A given segment corresponds to a particular naturally occurring protein if any 11 (or more) consecutive amino acids of the segment are found in exactly the same order in a portion of the naturally occurring protein. The segment is preferably less than 100 amino acids (less than 95 amino acids for the HPV strain 16 E7 protein) and more preferably less than 70 or less than 50 amino acids (e.g., 20-50). In one embodiment, it is less than 15. The segments within the polyepitope polypeptide can be arranged in any order within the polypeptide. The hybrid polypeptide preferably does not contain a sequence identical to the sequence of either full length, intact E6 or full length, intact E7 protein from HPV strain 16 or 18. The sequences of these proteins can be found at the web sites (ftp://ftp-t10.lanl.gov/pub/papilloma/SWISS-PROT-files/Human-papilloma/HPV 16.swp) and (ftp://ftp-t10.lanl.gov/pub/papilloma/SWISS-PROT-files/Human-papilloma/HPV 18.swp) as viewed on December 7, 1999.

The additional segments can be derived from any tumor antigen or naturally occurring antigenic protein of a pathogenic agent As used herein, "pathogenic agent" means a virus or microorganism that causes disease in a mammal. By "tumor antigen" is meant a protein or epitope which is expressed or in a tumor cell but not, or to a lesser degree, on a cell which is the non-tumor homolog of the tumor cell. Such tumor antigens frequently serve as markers for differentiating tumor cells from their normal counterparts. Examples of tumor antigens are listed in Table 1, such as a protein encoded by the Her2/neu gene, the prostate specific antigen gene, the melanoma antigen recognized by T cells (MART) gene, or the melanoma antigen gene (MAGE). Examples of proteins from pathogenic agents include proteins naturally expressed from the genome of a virus, e.g., a virus which chronically infects cells, such as human papillomavirus (HPV), human.immunodeficiency virus (HIV), herpes simplex virus (HSV), hepatitis B virus (HBV), or hepatitis C virus (HCV); a bacterium, such as mycobacteria, Helicobacter spp. e.g., *Helicobacter pylori,* or *Chlamydia* spp.; a fungus; or a parasitic eukaryote, such as *Plasmodium* species.

A representative list of class 1-binding epitopes of appropriate proteins, any of which could be included in the polyepitope polypeptides of the invention, is included in Table 2.

The hybrid polypeptide can contain a first epitope from an HPV protein and a second epitope that does not overlap with the first epitope and is from the same or a different HPV protein. ("Different HPV protein" can include a non-identical homolog of the first protein, derived from a different HPV strain than that from which the first protein was derived.) The first epitope binds to a first major histocompatibility complex (MHC) class I allotype and the second epitope binds to a second MHC class I allotype different from the first MHC class I allotype.

Because the hybrid polypeptide is made up of protein fragments from different proteins, orfrom non-contiguous fragments of a single protein, optionally linked by spacer amino acids or spacer sequences and optionally joined to a methionine residue or a targeting signal (see below), the sequence of this hybrid polypeptide is not identical to the amino acid sequence of either a naturally occurring protein or a fragment of a naturally occurring protein. Epitopes are included from, the HPV E6 or E7 protein from HPV strain 16 or 18, other HPV strains are 31, 33, 43, or 45. Thugs, the hybrid polypeptide includes, segments from each of the HPV strain 16 E6 and E7 proteins, and segments from each of the HPV strain 18 E6 and E7 proteins. The hybrid polypeptide is at least 236 amino acids in length, and can be; e.g., at least 250, or 300 amino acids.

The MHC class I allotypes to which the epitopes in the hybrid polypeptide bind can be any human class I allotypes, e.g., HLA-A1, HLA-A2, HLA-A3, HLA-A11, and/or HLA-A24. A given epitope may be promiscuous, i.e., bind more than one allotype.

The hybrid polypeptide may also include a third epitope of an HPV protein. This epitope, which can be derived from the same or a different HPV protein as the first and/or second epitope, binds to a third MHC class I allotype different from the first and second MHC class I allotypes. It may, of course, also bind to the first and/or second MHC class I allotype, in addition to the third. The hybrid polypeptide may include, e.g., at least 4, 5,6,7,8,9,10,15,25,35,40,50,60,80, or even 100 or more MHC class I allotype-binding epitopes from one or more HPV proteins. Some of these epitopes may overlap.

**TABLE 1: Tumor Antigens**

| Cancer | Associated Antigen |
|---|---|
| Melanoma | BAGE 2-10 |
| Breast/Ovarian | c-ERB2 (Her2/neu) |
| Burkitt's lymphoma/Hodgkin's lymphoma | EBNA-1 |
| Burkitt's lymphoma/Hodgkin's lymphoma | EBNA-2 |
| Burkitt's lymphoma/Hodgkin's lymphoma | EBNA-3 |
| Burkitt's lymphoma/Hodgkin's lymphoma | EBNA-3A |
| Burkitt's lymphoma/Hodgkin's lymphoma | EBNA-3C |
| Burkitt's lymphoma/Hodgkin's lymphoma | EBNA-4 |
| Burkitt's lymphoma/Hodgkin's lymphoma | EBNA-6 |
| Burkitt's lymphoma/Hodgkin's lymphoma | EBV |
| Burkitt's lymphoma/Hodgkin's lymphoma | EBV LMP2A |
| Melanoma | GAGE-1 |
| Melanoma | gp75 |
| Cervical | HPV 16 E6 |
| Cervical | HPV 16 E7 |
| Cervical | HPV 18 E6 |
| Cervical | HPV 18 E7 |
| Melanoma | MAG |
| Melanoma | MAGE-1 |
| Melanoma | MAGE-2 |
| Melanoma | MAGE-3 |
| Melanoma | MAGE-4b |
| Melanoma | MAGE-5 |
| Melanoma | MAGE-6 |
| Melanoma | MART-1/Melan-A |
| Pancreatic/Breast/Ovarian | MUC-1 |
| Melanoma | MUM-1-B |
| Breast/Colorectal/Buricitt's lymphoma | p53 |
| Melanoma | Pmel 17(gp100) |
| Prostate | PSA (Prostate Specific Antigen) |
| Melanoma | Tyrosinase |
| multiple | CEA (Carcinoembryonic Antigen) |
| lung | LRP (Lung Resistance Protein) |
| multiple | Bcl-2 |
| | Ki-67 |

**TABLE 2: Class I-associated Tumor and Pathogen Epitopes**

| Peptide | SEQ ID NO: | Source Protein |
|---|---|---|
| AARAVFLAL | 1 | BAGE 2-10 |
| YRPRPRRY | 2 | GAGE-19-16 |
| EADPTGHSY | 3 | MAGE-1 161-169 |
| SAYGEPRKL | 4 | MAGE-1 230-238 |
| EVDPIGHLY | 5 | MAGE-3161-169 |
| FLWGPRALV | 6 | MAGE-3271-279 |
| GIGILTV | 7 | MART-1 29-35 |
| ILTVILGV | 8 | MART-1 32-39 |
| STAPPAHGV | 9 | MUC-1 9-17 |
| EEKLIVVLF | 10 | MUM-1261-269 |
| MLLAVLYCL | 11 | TYROSINASE 1-9 |
| SEIWRDIDF | 12 | TYROSINASE 192-200 |
| AFLPWHRLF | 13 | TYROSINASE 206-214 |
| YMNGTMSQV | 14 | TYROSINASE 369-376 |
| KTWGQYWQV | 15 | PMEL 17 (GP100) 154-162 |
| ITDQVPFSV | 16 | PMEL 17 (GP100) 209-217 |
| YLEPGPTVA | 17 | PMEL 17(GP100) 280-288 |
| LLDGTATLRL | 18 | PMEL 17 (GP100) 476-485 |
| ELNEALELEK | 19 | p53 343-351 |
| STPPPGTRV | 20 | p53149-157 |
| LLPENNVLSPL | 21 | p53 25-35 |
| LLGRNSFEV | 22 | p53 264-272 |
| RMPEAAPPV | 23 | p53 65-73 |
| KIFGSLAFL | 24 | HER-2/neu 369-377 |
| IISAVVGIL | 25 | HER-2/neu 654-662 |
| CLTSTVQLV | 26 | HER-2/neu 789-797 |
| YLEDVRLV | 27 | HER-2/neu 835-842 |
| VLVKSPNHV | 28 | HER-2/neu 851-859 |
| RFRELVSEFSRM | 29 | HER-2/neu 968-979 |
| LLRLSEPAEL | 30 | PSA 119-128 |
| DLPTQEPAL | 31 | PSA 136-144 |
| KLQCVD | 32 | PSA 166-171 |
| VLVASRG RAV | 33 | PSA 36-45 |
| VLVHPOWVL | 34 | PSA 49-57 |
| DMSLLKNRFL | 35 | PSA 98-107 |
| QWNSTAFHQ | 36 | HBV envelope 121-130 |
| VLQAGFF | 37 | HBV envelope 177-184 |
| LLLCLIFL | 38 | HBV envelope 250-257 |
| LLDYQGML | 39 | HBV envelope 260-267 |
| LLVPFV | 40 | HBV envelope 338-343 |
| SILSPFMPLL | 41 | HBV envelope 370-379 |
| PLLPIFFCL | 42 | HBV envelope 377-385 |
| ILSTLPETTV | 43 | HBV core 529-538 |
| FLPSDFFPSV | 44 | HBV core 47-56 |
| KLHLYSHPI | 45 | HBV polymerase 489-498 |
| ALMPLYACI | 46 | HBVpolymerase 642-651 |
| HLYSHPIIL | 47 | HBV polym. 1076-1084 |
| FLLSLGIHL | 48 | HBV polym. 1147-1153 |
| HLLVGSSGL | 49 | HBV polymerase 43.51 |
| GLSRYVARL | 50 | HBV polymerase 455-463 |
| LLAQFTSAI | 51 | HBV polymerase 527-535 |
| YMDDVVLGA | 52 | HBV polymerase 551-559 |
| GLYSSTVPV | 53 | HBV polymerase 61-69 |
| NLSWL | 54 | HBV polymerase 996-1000 |
| KLPQLCTEL | 55 | HPV 16 E6 18-26 |
| LQTTIHDII | 56 | HPV 16 E6 26-34 |
| FAFRDLCIV | 57 | HPV 16 E6 52-60 |
| YMLDLQPET | 58 | HPV 16 E711-19 |
| TLHEYMLDL | 59 | HPV 16 E7 7-15 |
| LLMGTLGIV | 60 | HPV 16 E7 82-90 |
| TLGIVCPI | 61 | HPV 16 E7 86-93 |
| LLMGTLGIVCPI | 62 | HPV 16 E7 82-93 |

Unless otherwise specified, the epitopes in the hybrid polypeptides of the invention may overlap to some degree, e.g., the first epitope may overlap (i.e., share at least one amino acid residue, and share up to all but one residue) with the third epitope, or they may be non-overlapping.

The polypeptide includes a targeting signal. A targeting signal is a peptide which directs intracellular transport or secretion of a peptide to which it is attached. The targeting signal can be at the amino terminus, e.g., a signal sequence, or carboxy terminus, or within the hybrid polypeptide, so long as it functions in that site.

The targeting signal can be any recognized signal sequence, e.g., a signal sequence from the adenovirus E3 protein. A preferred targeting signal is the signal peptide of HLA-DRα: Met Ala Ile Ser Gly Val Pro Val Leu Gly Phe Phe Ile Ile Ala Val Leu Met Ser Ala Gln Glu Ser Trp Ala (SEQ ID NO:63).

The targeting signal may optionally be modified to introduce an amino acid substitution at the junction(s) between the targeting signal and the adjacent segment(s) to promote cleavage of the targeting sequence from the epitopes by, erg., a signal peptidase.

Any of the segments within the hybrid polypeptide may be separated from the others by a spacer amino acid or a spacer sequence.

The nucleic acid of the invention may encode a hybrid polypeptide including a first epitope and a second epitope, wherein the first epitope is from a first HPV protein and the second epitope is from a second HPV protein different from the first HPV protein, provided that the sequence of the second epitope does not occur within the first HPV protein, i.e., they are necessarily derived from different proteins. The different proteins can be from the same or different strains of H PV, e.g., types 16 and 18. The hybrid polypeptide can, of course, include additional epitopes from the same or different HPV proteins, or from other pathogens or tumor antigens.

Also within the invention is a nucleic acid encoding a hybrid polypeptide including a plurality (e.g., at least 2, 3, 4, 5, 6, 7, 8, 9, 10, 15, 20, or 30) MHC class binding epitopes from an HPV protein. The sequence of the entire hybrid polypeptide is not identical to the sequence of either a naturally occurring HPV protein or a fragment of a naturally occurring HPV protein, by virtue of sequence insertions, internal deletions, or substitutions, accomplished, e.g., by genetic engineering techniques.

The nucleic acid encodes a hybrid polypeptide that includes a plurality of HLA-binding epitopes from an HPV strain 16 E6 protein, a plurality of HLA-binding epitopes from an HPV, strain 16 E7 protein, a plurality of HLA-binding epitopes from an HPV strain 18 E6 protein, and a plurality of epitopes from an HPV strain 18 E7 protein. Each plurality of epitopes can include an epitope selected from the group consisting of an HLA-A1-binding epitope, an HLA-A2-binding epitope, an HLA-A3-binding epitope, an HLA-A11-binding epitope, and an HLA-A24-binding epitope, and preferably at least two or three selected from this group. The members of each plurality of epitopes are different from the members of each of the other plurality of epitopes. Each plurality of epitopes may include at least two HLA-A1-binding epitopes, at least two HLA-A2-binding epitopes, at least two HLA-A3-binding epitopes, at least two HLA-A11-binding epitopes, and/or at least two HLA-A24-binding epitopes. A given plurality can be distributed on more than one segment.

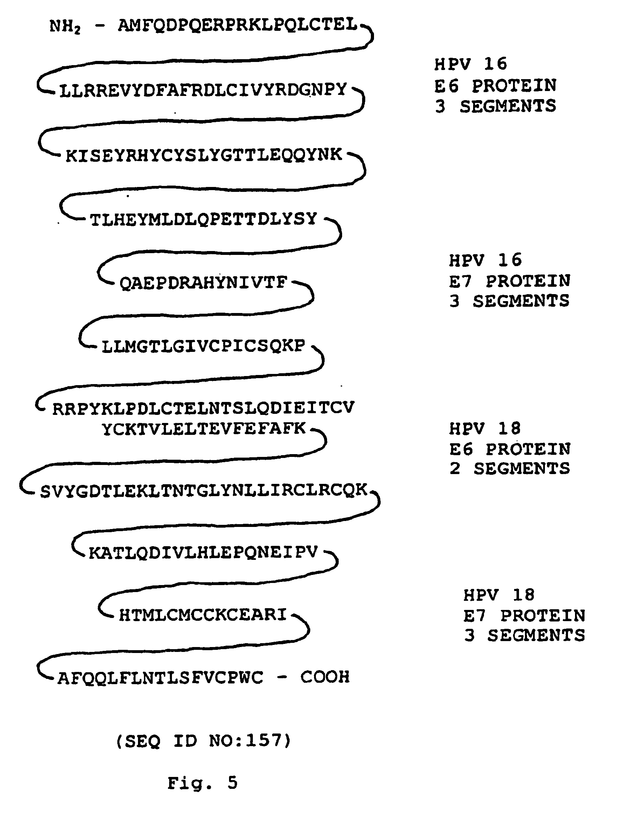

The nucleic acid of the invention encodes a hybrid polypeptide comprising the peptide segments of Fig. 5 (SEQ ID NO: 157) and a signal sequence and does not comprise a sequence identical to the sequence of either full length, intact E6 or full length intact E7 protein from HPV strain 16 or 18.
The polypeptide includes the segments.
AMFQDPQERPRKLPQLCTEL (SEQ ID NO:64), LLRREVYDFAFRDLCIVYRDGNPY (SEQ ID NO:65), KISEYRHYCYSLYGTTLEQQYNK (SEQ ID NO:66), TLHEYMLDLOPETTDLYSY (SEQ ID NO:67), QAEPDRAHYNIVTF (SEQ ID NO: 68), LLMGTLGlVCPlCSQKPlSEQ ID NO:69), RRPYKLPDLCTELNTSLQDIEITCVYCKTVLELTEVFEFAFK (SEQ ID NO: 152), SVYGDTLEKLTNTGLYNLLIRCLRCQK (SEQ ID NO:153), KATLQDIVLHLEPONEIPV (SEQ ID NO:154), HTMLCMCCKCEARI (SEQ ID NO:155), and AFQQLFLNTLSFVCPWC (SEQ **ID** NO:156). The segments can be processed to produce multiple epitopes. The hybrid polypeptide can include the peptide segments of Fig. 5, as well as additional HPV E6 or E7 sequence. Most preferably, the segment is less than 100, 70, 50, 40, 30, or 20 amino acids. The hybrid polypeptide does not contain a sequence identical to the sequence of either full length, intact E6 or full length, intact E7 protein from HPV strain 16 or 18.

The nucleic acids described above can be provided in a plasmid, bacterial, or viral vector. When the nucleic acids are used *in vivo,* it is preferable to use plasmid vectors containing the above-described nucleic acids. The vector is preferably an expression vector which contains one or more regulatory sequences (which could include a promoter) which permit expression in a cell of interest The regulatory sequence(s) are operatively linked to the sequence encoding the hybrid polypeptide, such that they drive expression of the latter.

A cell into which the nucleic acid of the invention has been introduced (e.g., by transfection or infection) in the form of the plasmid, bacterial, or viral vector, either transiently or stably can be, e.g., a B cell, dendritic cell (DC), Langerhanscell, orotherantigen presentingcell (APC). The cell may be cultured or otherwise maintained underconditions permitting expression of the hybrid polypeptide from the nucleic acid, e.g., the plasmid, encoding it.

As used herein, "isolated DNA" covers both (1) a DNA the full sequence of which does not occur within any naturally occurring DNA, and (2) a DNA the full sequence of which does occur within a naturally occurring DNA, but which is free of the genes that flank that sequence in the genome of the organism in which that sequence naturally occurs. The term therefore includes a recombinant DNA incorporated into a vector, into an autonomously replicating plasmid or virus, or into the genomic DNA of a prokaryote or eukaryote. It also includes a separate molecule such as a cDNA. a genomic fragment, a fragment produced by polymerase chain reaction (PCR), or a restriction fragment.

The invention further includes hybrid polypeptides encoded by the above-described nucleic acids. The hybrid polypeptides described herein may optionally include an amino terminal methionine residue. The hybrid polypeptides described herein also may optionally include a GLAG (or other monoclonal antibody determinant) or an antibody recognition site (e.g., a myc or his tag) located directly 3' of the last epitope coding sequence. Also included in the hybrid polypeptide are segments, as discussed above. At least some of the segments correspond to portions of one or more naturally occurring proteins. They may be separated by spacer amino acids or spacer peptides.

The hybrid polypeptide described herein can be a substantially pure polypeptide. "Substantially pure polypeptide" covers both (1) a polypeptide the full sequence of which is not identical to that of any naturally occurring polypeptide, and (2) a polypeptide which does have the sequence of a naturally occurring polypeptide, but which is separated from those components (proteins and other naturally-occurring organic molecules) which naturally accompany it in the biological context (e.g., cell) in which it naturally occurs. Typically, the polypeptide is substantially pure when it constitutes at least 60%, by weight, of the protein in the preparation. Preferably, at least 75%, more preferably at least 90%, and most preferably at least 99%, by weight, of the protein in preparation is the polypeptide.

The nucleic acids (e.g., viral, bacterial, or plasmid vectors) or hybrid polypeptides can optionally be provided in a microparticle that also includes a polymeric matrix. In preferred embodiments, the polymeric matrix consists essentially of poly-lactic acid (PLA), poly-glycolic acid (PGA), or a copolymer of poly-lactide-*co*-glycolide acid (PLGA). The microparticle preferably has a diameter of, e.g., 0.02 to 20 microns, or less than about 11 microns. A plurality of the microparticles preferably has an average diameter of, e.g., 0.02 to 20 microns, less than about 11 microns, or less than about 5 microns.

The nucleic acids and hybrid polypeptides described herein can alternatively be incorporated into liposomes or immune-stimulating complexes (ISCOMS) or delivered with naturally occurring polymers, synthetic polymers, biopolymers, cationic lipids, condensing agents, dendrimers, other biomaterials, oil-containing adjuvants, and other adjuvants such as QS21 or saponin. The nucleic acids and hybrid polypeptides described herein may alternatively be administered using any other suitable delivery vehicle known in the art, or can be delivered without a delivery vehicle (other than aqueous solution), e.g., "naked DNA".

The invention in addition includes a therapeutic composition containing the above-described nucleic acids or polypeptides, a pharmaceutically acceptable carrier, and optionally, one of the above-discussed delivery vehicles.

Also provided is a method of eliciting an immune response (e.g., an antibody response or a cellular immune response, including an MHC class 1-mediated or class 11-mediated immune response) in a mammal by administering the above-described nucleic acids or hybrid polypeptides to the mammal. Preferably, the mammal bears at least one MHC class I or II allotype which binds to an epitope derived from the polyepitope polypeptide. The mammal can be, e.g., a human, non-human primate, dog, cat, rabbit, cow, horse, sheep, pig, goat, mouse, rat, guinea pig, or hamster.

When at least one of the epitopes is an HPV epitope, appropriate subjects for the method include, e.g., a human who suffers from, or is at risk of, condyloma, e.g., exophytic condyloma, flat condyloma, cervical cancer, other HPV-associated-cancers diseases or conditions, respiratory papilloma, conjunctival papilloma, genital-tract or anal-tract HPV infection, or cervical dysplasia. The nucleic acid or hybrid polypeptide, or delivery vehicle containing one or more of these compositions, can be administered directly to a mucosal tissue, e.g., vaginal, nasal, lower respiratory, ocular, or applied to gastrointestinal (e.g., rectal, cervical, or dermal) tissue, of the mammal. Alternatively, the nucleic acid or hybrid polypeptide, or delivery vehicle containing the same, can be administered systemically: for example, intravenously, intramuscularly, intradermally, orally, subcutaneously, intraarterially, intraperitoneally, or intrathecally.

By "spacer amino acid" is meant a single residue inserted between two neighboring segments. ("A" and "B". in that order) in a polypeptide of the invention, where the residue is different from the amino acid which flanks the carboxy terminus of A and also is different from the amino acid which flanks the amino terminus ofB in the respective full length proteins from which A and B were derived ("X" and "Y", respectively). Thus, the spacer amino acid forms a point of discontinuity from the X-derived sequence of A and the Y-derived sequence of B, in the polypeptide of the invention. Typically, the amino acid will be one of the twenty naturally occurring amino acids, e.g., Ala, Leu, Ile, or Gly, and in general can be any amino acid except (1) the one that naturally flanks the carboxy terminus of A in protein X, and (2) the one that naturally flanks the amino terminus of B In protein Y.

By "spacer sequence" is meant a sequence of two or more amino acid inserted between two neighboring segments, e.g., "A" and "B", in a polypeptide of the invention. The sequence of the spacer is different from the sequences which flank the carboxy terminus of A and the amino terminus of B in the respective full length proteins ("X" and "Y") from which A and B were derived. Thus, the spacer sequence forms a point of discontinuity from both the X-derived sequence of A and the Y-derived sequence of B in the polypeptide of the invention.

Examples of spacer sequences include Ala Ala, Ala Leu, Leu Leu, Leu Ala, Leu Ile, Ala Ala Ala, Ala Gly Leu, Phe Ile Ile, etc. Generally, the spacer sequence will include nonpolar amino acids, though polar residues such as Glu, Gin, Ser, His, and Asn could also be present, particularly for spacer sequences longer than three residues. The only outer limit on the total length and nature of each spacer sequence derives from considerations of ease of synthesis, proteolytic processing, and manipulation of the polypeptide and/or nucleic acid It Is generally unnecessary and probably undesirable to use spacer sequences longer than about four or five residues, though they could be, for example, up to 6, 8, 10, 15, 20, 30, or 50 residues. Of course, they could be even longer than 50 residues.

Spacer amino acids and spacer sequences are useful for promoting processing to release epitopes. The spacers are typically removed from the polypeptide by proteolytic processing in the cell, along with any sequence between epitopes within a given segment. This leaves the epitopes intact for binding to MHC molecules or (upon secretion from the cell) antibodies. Occasionally a spacer amino acid or part of a spacer sequence will remain attached to an epitope through incomplete processing. This generally will have little or no effect on binding to the MHC molecule.

An advantage of the invention is that it permits delivery of MHC class 1 or class II-binding epitopes from polypeptides having only a partial sequence of a protein of a pathogen or tumor antigen. Thus, problems associated with interference of antigen presentation by viral proteins; or deleterious effects seen in over-expression of particular viral proteins or tumor antigens, are avoided.

Another advantage of the invention is that the nucleic acid sequences, or delivery vehicles containing the nucleic acid sequences, may promote associated immune responses, e.g., IL-12 and γ-interteron (IFN) release from macrophages, NK cells, and T cells. For instance, phagocytosis of microspheres containing DNA in general can promote TNF and IFN secretion by human APC.

A further advantage of the invention Is that the assortment of epitopes within the polyepitope polypeptides described herein increases the likelihood that at least one epitope will be presented by each of a variety of HLA allotypes. This allows for immunization of a population of individuals polymorphic at the HLA locus, using a single hybrid polypeptide or nucleic acid encoding the polypeptide. The hybrid polypeptide can be specific for a particular pathogen, including two or more strains of the same pathogen, or can contain epitopes derived from two or more pathogens. In addition, the hybrid polypeptide can be specific for a particular tumor antigen, or can contain epitopes derived from two or more tumor antigens.

Unless otherwise defined, all technical and scientific terms used herein have the same meaning as commonly understood by one of ordinary skill in the art to which this invention belongs. Suitable methods and materials are described below, although methods and materials similar or equivalent to those described herein can also be used in the practice or testing of the present invention.

In addition, the materials, methods, and examples are illustrative only and not intended to be limiting.

Other features and advantages of the invention will be apparent from the following detailed description, and from the claims,

### Brief Description of the Drawings

Fig. 1 is a schematic drawing of a polyepitope polypeptide which includes four segments, each segment including multiple epitopes.
Fig. 2 is a schematic drawing of a polyepitope polypeptide including the amino acid sequences of segments derived from the HPV strain 16 E6 and E7 proteins.
Fig. 3 is a graph illustrating the results of an *in vitro* assay In which human T cells stimulated with the peptide HPV 16E7 44-52 were tested against HLA-A1/A3-expressing targets infected with either wildtype vaccinia vector (open circles) or vaccinia vector encoding HPV 16E7 44-52 as part of an HPV 16 E6/E7 polyepitope polypeptide.
Fig. 4 is a schematic drawing of a polyepitope polypeptide including the amino acid sequences of segments derived from the HPV strain 18 E6 and E7 proteins.
Fig. 5 is a schematic drawing of a polyepitope polypeptide including the amino acid sequences of segments derived from the E6 and E7 proteins of HPV strains 16 and 18.
Fig. 6 is a graph illustrating the results of an in vitro assay in which T cells from DNA-immunized mice were expanded *In vitro* by exposure to peptide 124 or peptide 272 and subsequently tested against target cells infected with wildtype vaccinia vector, vaccinia vector encoding HPV Dra 16/18, or vaccinia vector encoding HPV 16/18.

### Detailed Description

The present invention provides nucleic acids encoding polyepitope polypeptides which include multiple immunogenic segments, each segment containing one or more MHC class I or II-restricted epitopes, or both. Nucleic acids and polypeptides of the invention are useful for generating or enhancing prophylactic or therapeutic immune responses against pathogens, tumors, or autoimmune disease in a population of individuals having diverse MHC allotypes. In addition, the nucleic acids and polypeptides of the invention are also useful as positive controls in T cell stimulation assays *in vitro,* and as tools to understand processing of epitopes within cells.

One or more of the segments present within the polyepitope polypeptide can be processed into multiple distinct epitopes to form either MHC class I-binding epitopes, which are typically associated with cytotoxic T cell (CTL) immune responses, or MHC class II-binding epitopes, which are typically associated with helper T cell immune responses. Other epitopes can be B cell epitopes which stimulate production of epitope-specific antibodies.

Included In the invention are nucleic acids encoding polyepitope polypeptides useful for preventing or treating HPV-associated infections, particularly infections associated with warts, cervical or anal dysplasia, cervical cancer, and other HPV-assodated cancers. For example, the polypeptides and nucleic adds of the invention can be used as vaccines prophylactically or therapeutically in subjects known to be infected by HPV, suspected of being infected by HPV, or likely to become infected by HPV. Other suitable subjects include those displaying symptoms of, or likely to develop, HPV-associated conditions. The immunogenic polypeptides, and nucleic acids encoding these polypeptides, can also be used as vaccines for preventing or treating conditions associated with infections of HPV strain 16, e.g., bowenoid papulosis, anal dysplasia, respiratory or conjunctival papillomas, cervical dysplasia, cervical cancer, vulval cancer, or prostate cancer. They can also be used to treat conditions associated with other HPV strains, especially those associated with HPV strains 6, 11, 18, 31, 33, 35, and 45. These conditions include, e.g., exophytic condyloma (HPV strains 6 and 11), flat condyloma, especially of the cervix (HPV strains 6, 11, 16, 18, and 31 giant condyloma (HPV strains 6 and 11), cervical cancer (HPV strains 18, 31, and 33, in addition to HPV strain 16), respiratory and conjunctival papillomas (HPV 6 and 11), and infection with genital-tract HPVs (HPV 6, 11, 16, 18, 31, 33, and 35).

### Identification of MHC-binding epitopes from HPV proteins

For an epitope to generate effective cytotoxic T cell (CTL) responses, it must bind to an MHC molecule on an antigen-presenting cell (APC), and the resulting receptor-ligand complex must be recognized by a T cell receptor expressed on the CTL. Epitopes which bind to a specific MHC allele can be identified by first synthesizing a series of overlapping peptide fragments from a protein of interest, e.g., an HPV protein such as HPV E6 or E7, and testing the peptides in art-recognized binding studies with a radiolabeled peptide known to bind to the MHC allele. If a test peptide demonstrates specific binding to an MHC allele as measured by, for example, competition with the radiolabeled test peptide (i.e., it is an epitope), the epitope can be combined with additional epitopes (overlapping or adjacent) to produce or define a segment.

Alternatively, epitopes can be identified by refolding soluble MHC molecules in the presence of radiolabeled β2-microglobulin and a test peptide. The complete complex will refold and produce a receptor of the correct size. β2-microglobulin dissociates from the complex at a measurable rate that is directly correlated with the binding affinity of the test peptide (Garboczi et al., Proc. Nat. Acad. Sci. USA 89:3429-33, 1992; Parker et al., J. Biol. Chem. 267:5451-5459, 1992; and Parker et al., J. Immunol. 149: 1896-1904, 1992). Analysis of this type of data has resulted in an algorithm that predicts the dissociation times of a given test peptide for an HLA-A2 receptor (Parker et al., J. Immunol. 152:163-175, 1994). Fast dissociation has been correlated with low affinity, and slow dissociation with high affinity. This algorithm has been expanded and is available for predicting binding affinity of epitopes for the HLA-A allotypes, -A1, -A2, -A3, -A11, and -A24. The algorithm can be found at the web site (http://wwwbimas.dcrt.nih.gov/mo)bio/hla_bind/index.html). Most of the HPV 16 E6 and E7 epitopes that are known in the art to bind one of the five listed HLA-A receptors are predicted by this algorithm to cause β-microglobulin dissociation times of greater than 2 h.

Epitope binding can also be determined using previously identified epitopes derived from HPV 16 E6 and E7 (Kast et al., J. Immunol. 152:3904-12, 1994). Additional binding studies on other HPV putative epitopes, e.g., HPV 18 E6-and E7- derived epitopes, are performed using overlapping 8, 9, or 10-mers from the HPV 18 E6 and E7 proteins in binding assays involving each of the five HLA-A allotypes, using one of the above-described protocols.

Alternatively, epitopes may be identified by identifying MHC class I or class II-binding peptides using techniques described in e.g., U.S. Pat. No. 5,827,516, and USSN 09/372,380.

### T cell assays for epitopes

Epitopes which bind *in vitro* to MHC molecules as described above can be analyzed for their effectiveness at stimulating human T cell-responses in an *in vitro* immunization assay. The assay has been used previously to identify human and murine T cell-responsive epitopes, including several that are derived from HPV proteins (Alexander et al., Amer. J. Obstet, and Gynecol. 1-75:1586-1593, 1996; Tarpey et al., Immunology 81:222-27, 1994). These assays have also been used to generate large numbers of specific CTL for immunotherapy (Tsai et al., Crit. Rev. Immunol. 18:65-75, 1998). To ensure reliability, it is desirable to perform the first round of T cell stimulation in the presence of dendritic cells (DCs) pulsed with the test peptide. Moreover, inclusion of IL-10 during the stimulation may suppress the non-specific responses that may sometimes arise during culture of the cells. T cell activation may then be examined using an ELISA assay to measure γ-IFN secretion, or by use of FACS to determine the increase in CD8+, CD 16- cells containing γ-IFN by tricolor analysis. Alternatively, T cell activation can be measured using a ⁵¹Cr release CTL assay or a tetramer-based assay.

It is possible that not every individual with a given allotype will respond to a particular epitope. For example, one individual whose cells bear the HLA-A2 allotype may respond to a given epitope, whereas a second such individual may not. One reason for this differential responsiveness may be that those individuals who have been infected with HPV previously have a higher precursor T cell frequency than a naive individual. Another may be related to differences in the T cell repertoire between the two individuals. To overcome this difficulty. T cells from two donors, and even more preferably three donors, for each HLA allotype can be tested. For the more common alleles (i.e., HLA-A2 and -A3) up to four donors are preferably tested.

Each epitome is tested initially with cells from one donor. If an epitopes does not stimulate a T cell response using cells of the first donor, It is tested again with cells from a second donor, and then a third donor. If the epitope does not demonstrate T cell reactivity after two or three attempts, it is preferably not represented in the polypeptides of the invention.

Altering the method by which the *in vitro* presentation of antigen is performed may enhance analysis. An initial stimulation ofT cells with DCs is typically part of the *in vitro* immunization. To enhance the immunization, DCs can be added at each round of stimulation to ensure adequate antigen presentation and T cell stimulation, e.g., using previously generated and subsequently frozen DCs. Leukopaks or blood samples can be obtained from individuals with high grade squamous intraepithelial lesions (HSIL) or cervical cancer. These individuals may have been primed *in vivo* and have increased numbers of T cells in their blood.

Alternatively, the epitopes can be selected for inclusion in the construct based solely on their binding affinity to HLA molecules, or identified based upon analysis of naturally processed peptides, as described herein and in Chicz et al., J. Exp. Med. 178:27-47, 1993, and U.S. Patent 5,827,516.

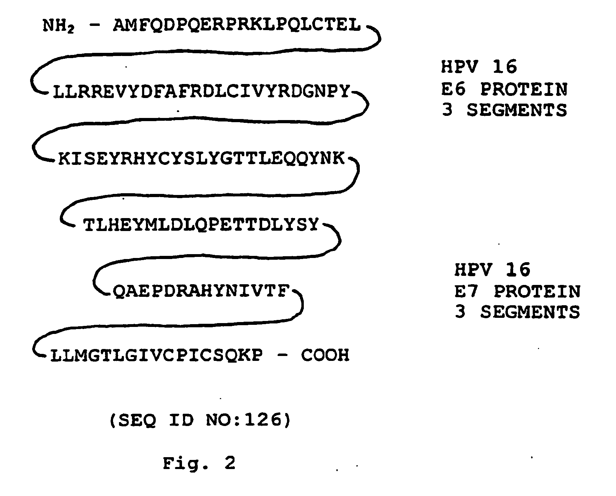

The amino acid sequence of a hybrid polypeptide (SEQ ID NO: 126) based on sequences found in the HPV strain 16 E6 and strain 16 E7 proteins is shown in Fig. 2. The hybrid polypeptide is created by fusion of fragments of the E6 protein (amino acids 7-26 (SEQ ID NO:64), 44-67 (SEQ ID NO:65), and 79-101 (SEQ ID NO:66)) and the E7 protein (amino acids 7-25 (SEQ ID NO:67), 44-57 (SEQ **ID** NO:68), and 82-98 (SEQ **ID** NO:69)). To prevent retinoblastoma (Rb) protein binding to the hybrid polypeptide, amino acids 26 and 27 of the E7 protein are not included, In addition, the cysteine at amino acid 24 has been converted to a serine residue. In the parlance defined above, this renders that serine residue, together with residue 25, a "spacer sequence" between segments corresponding to E7 protein residues 7-23 and 44-57, respectively.

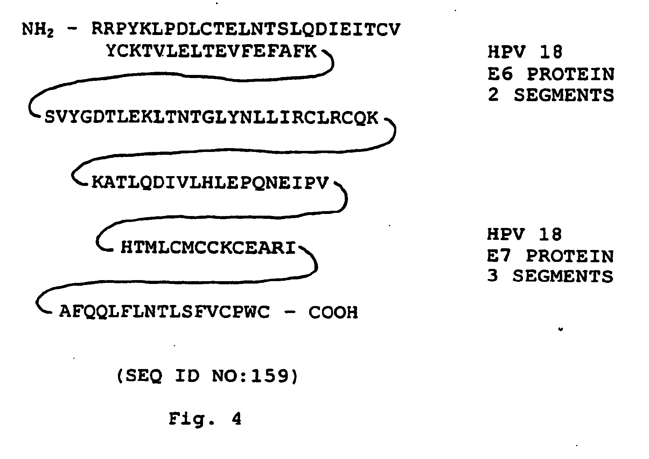

The amino acid sequence of a second hybrid polypeptide (SEQ ID NO:159) based on sequences found in the HPV strain 18 E6 and strain 18 E7 proteins is shown In Fig. 4. The hybrid polypeptide Is created by fusion of fragments of the E6 protein (amino acids 9-50 (SEQ ID NO:152) and 84-110 (SEQ ID NO: 153)), and the E7 protein (amino acids 5-23 (SEQ ID NO:154), 59-72 (SEQ ID NO:155), and 85.101 (SEQ ID NO: 156)).

The amino acid sequence of a third hybrid polypeptide (SEQ ID NO:157) based on sequence found In the E6 and E7 proteins of HPV strains 16 and 18 is shown in Fig. 5. The hybrid polypeptide is created by fusion of fragments of the HPV strain 16 E6 protein (amino acids 7-26 (SEQ ID NO:84), 44-67 (SEQ ID NO:65), and 79-101 (SEQ ID NO: 66)), the HPV strain 16 E7 protein (amino acids 7-25 (SEQ ID NO:67), 44-57 (SEQ ID NO:68), and 82-98 (SEQ ID NO: 69)), the HPV strain 18 E6 protein (amino acids 9-50 (SEQ ID NO: 152) and 84-110 (SEQ ID NO:153)), and the HPV strain 18 E7 protein (amino acids 5-23 (SEQ ID NO:154), 59-72 (SEQ ID NO:155), and 85-101 (SEQ ID NO:156)). As described above with respect to the hybrid polypeptide of SEQ ID NO:128, those amino acids of the HPV strain 16 E7 protein that would cause the polypeptide to bind to the Rb protein are excluded. Furthermore, an inserted spacers sequence identical to that present in SEQ ID NO:125 lies between the segments corresponding to residues 7-23 and 44-57, respectively.

Any of the hybrid polypeptides described herein may optionally include an initiator methionine, a leader sequence, or any targeting sequence (e.g., a transmembrane domain). The terms "targeting sequence" and "trafficking sequence" are used Interchangeably herein. For example, when the third hybrid polypeptide has a methionine added to its amino terminus, it has the amino acid sequence of SEQ ID NO:158. Furthermore, when the third hybrid polypeptide has an HLA-DRα leader sequence added to its amino terminus, it has the amino acid sequence of SEQ ID NO:160.

Table 3 lists other MHC-binding epitopes from the HPV strain 16 E8 and E7 and the HPV strain 18 E6 and E7 polypeptides. Any of these epitopes can be incorporated into a hybrid polyepitope of the invention..

**TABLE 3: HPV E6 and E7 MHC-binding epitopes**

| **HPV 16 E6 Peptides** | | **SEQ ID NO** |
|---|---|---|
| A1 - binding | | |
| | ISEYRHYCY | 70 |
| | FQDPQERPR | 71 |
| | RREVYDFAF | 72 |
| | TTLEQQYNK | 73 |
| | FQDPQERPRK | 74 |
| | ISEYRHYCYS | 75 |
| | KISEYRHYCY | 76 |
| | GTTLEQQYNK | 77 |

| **A2 - binding** | | |
|---|---|---|
| | KLPQLCTEL | 55 |
| | KISEYRHYC | 78 |
| | FAFRDLCIV | 57 |
| | YCYSIYGTTL | 79 |
| | SEYRHYCYSL | 80 |

| **A3 - binding** | | |
|---|---|---|
| | AMFQDPQER | 81 |
| | LLRREVYDF | 82 |
| | TTLEQQYNK | 73 |
| | IVYRDGNPY | 83 |
| | KLPQLCTEL | 55 |

| **A11 - binding** | | |
|---|---|---|
| | TTLEQQYNK | 73 |
| | GTTLEQQYNK | 77 |

| **A24 - binding** | | |
|---|---|---|
| | VYDFAFRDL | 84 . |
| | CYSLYGTTL | 85 |

| **HPV 16 E6 Peptides** | | **SEQ ID NO** |
|---|---|---|
| | EYRHYCYSL | 86 |
| | KLPQLCTEL | 55 |
| | DPQERPRKL | 87 |
| | HYCYSLYGT | 88 |
| | DFAFRDLCI | 89 |
| | LYGTTLEQQY | 90 |
| | HYCYSLYGTT | 91 |
| | EVYDFAFRDL | 92 |
| | EYRHYCYSLY | 93 |
| | VYDFAFRDLC | 94 |
| | YCYSIYGTTL | 79 |

| **HPV 16 E7 Peptides** | | **SEQ ID NO** |
|---|---|---|
| **A1 - binding** | | |
| | QAEPDRAHY | 95 |
| | IVCPICSQK | 96 |
| | QPETTDLY | 97 |
| | QAEPDRAHYN | 98 |
| | DLQPETTDLY | 99 |

| **A2 - binding** | | |
|---|---|---|
| | YMLDLQPET | 58 |
| | TLHEYMLDL | 59 |
| | LLMGTLGIV | 60 |
| | LMGTLGIVC | 100 |
| | MLDLQPETT | 101 |
| | TLGIVCPIC | 102 |
| | DLQPETTDL | 103 |
| | GTLGIVCPI | 104 |
| | YMLDLOPETT | 105 |
| | LQPETTDLY | 106 |
| | LLMGTLGIVC | 107 |

| **A3 - binding** | | |
|---|---|---|
| | TLHEYMLDL | 59 |
| | IVCPICSQK | 96 |

| **A11 - binding** | | |
|---|---|---|
| | IVCPICSQK | 96 |

| **A24 - binding** | | |
|---|---|---|
| | DLQPETTDL | 103 |
| | TLHEYMLDL | 59 |
| | TPTLHEYML | 108 |
| | RAHYNIVTF | 109 |
| | GTLGIVCPI | 104 |
| | EPDRAHYNI | 110 |

| **HPV 18 E6 Peptides** | | **SEQ ID NO** |
|---|---|---|
| **A1 - binding** | | |
| | LTEVFEFAFK | 127 |

| **A2 - binding** | | |
|---|---|---|
| | KLPDLCTEL | 124 |
| | GLYNLLIRC | 128 |
| | SLQDIEITC | 129 |

| **HPV 16 E7 Peptides** | | **SEQ ID NO** |
|---|---|---|
| **A1 - binding** | | |
| | SLQDIEITCV | 130 |
| | LQDIEITCV | 131 |
| | KTVLELTEV | 132 |
| | ELTEVFEFA | 133 |
| | KLTNTGLYNL | 134 |
| | LTNTGLYNL | 135 |
| | GLYNLLIRCL | 125 |

| **A3- binding** | | |
|---|---|---|
| | VLELTEVFEF | 136 |
| | SVYGDTLEK | 137 |
| | LLIRCLRCOK | 138 |

| **A11 - binding** | | |
|---|---|---|
| | CVYCKTVLEL | 139 |
| | SVYGDTLEK | 137 |
| | LLIRCLRCQK | 138 |

| **A24 - binding** | | |
|---|---|---|
| | VYCKTVLEL | 140 |
| | VYGDTLEKL | 141 |
| | LTNTGLYNLL | 142 |

| **HPV 18 E7 Peptides** | | **SEQ ID NO** |
|---|---|---|
| **A1 - binding** | | |
| | HLEPONEIPV | 143 |

| **A2 - binding** | | |
|---|---|---|
| | TLQDIVLHL | 144 |
| | ATLQDIVLHL | 145 |
| | QLFLNLSFV | 146 |
| | MLCMCCKCEA | 147 |
| | CMCCKCEARI | 148 |
| | FQQLFLNTL | 149 |
| | TLSFVCPWC | 150 |

| **A3 - binding** | | |
|---|---|---|
| | HTMLCMCCK | 122 |

| **A11 - binding** | | |
|---|---|---|
| | HTMLCMCCK | 122 |

| **A24 - binding** | | |
|---|---|---|
| | QLFLNTLSF | 123 |
| | FQQLFLNTL | 149 |
| | AFQQLFLNTL | 151 |

### Nucleic acids encoding polypeptides containing multiple HPV epitopes

Nucleic acids encoding polyepitope polypeptides containing epitopes identified as described above can be generated using standard techniques, e.g., by overlapping PCR or linking of oligonucleotides. Preferably, codons are selected for inclusion in the construct to optimize production of the polyepitope polypeptides in bacterial or mammalian systems.

Different segments in the encoded polyepitope polypeptide, e.g., segments derived from different proteins, or from non-adjacent regions of the same protein, can be checked using a sequence analysis program; e.g., the BLAST program, to determine if the amino acid sequences ar the junctions of the segments show inadvertent similarity to known human proteins. If homologies exist, the polypeptide can be redesigned to alter the order of the epitopes in the polypeptide to eliminate the regions of homology. If a particular epitope itself is over 75% identical to a portion of a known human protein, it is preferably not included in the polyepitope polypeptide.

The order of the segments within a given polyepitope polypeptide can correspond to the order in which the segments appear in the native protein, though some of the amino acid sequence between (i.e., at least one residue) the individual segment in the native protein may be deleted. Alternatively, the segment order may differ from that in the naturally occurring protein. The latter arrangement is preferable if a natural alignment results in a polypeptide having regions of homology to a human protein.

For HPV-derived epitopes, the nucleic acid construct preferably does not encode an epitope which contains the known retinoblastoma protein (Rb)-binding site in the HPV E7 protein. The construct can encode, e.g., a polypeptide which includes segments from each of the HPV 16 E6, HPV 16 E7, HPV 18 E6, and HPV 18 E7 proteins, where each segment contains epitopes for binding to one or more of the HLA alleles HLA-A1, HLA-A2, HLA-A3, HLA-A11, and HLA-A24. This corresponds to about 12-20 epitopes per HLA allele, for about 60-100 epitopes in the polyepitope polypeptide.

Regulatory elements can be included in the construct to facilitate expression of the nucleic acid encoding the polyepitope polypeptide. These elements include sequences for enhancing expression in human or other mammalian cells, e.g., promoters, RNA stabilization sequences 5' and/or 3' to the coding sequence, introns (which can be placed at any location within or adjacent to the encoded sequence), and poly(A) addition sites, as well as an origin of replication and one or more genes encoding selectable markers enabling the constructs to replicate and be selected in prokaryotic and/or eukaryotic hosts. A T7 polymerase promoter or other type of promoter (e.g., a tissue-specific promoter such as a muscle-specific promoter, or a cell-specific promoter such as an APC-specific promoter) is optionally present at the 5' end of the coding sequence, and a sequence encoding a FLAG or other mAb determinant is optionally present directly 3' of the last epitope coding sequence. The construct may also contain other transcriptional and translational signals, such as a Kozak sequence.

The construct may in addition include a sequence encoding a targeting signal that directs the polyepitope polypeptide to a desired intracellular compartment, the targeting signal being linked to the polypepitope polypeptide. Targeting signals can direct the polyepitope polypeptide to endoplasmic reticulum (ER), the golgi, the nucleus, a lysosome, a class II peptide loading compartment, or an endosome, and include signal peptides (the amino terminal sequences which direct proteins into the ER during translation), ER retention peptides such as KDEL (SEQ ID NO:111), and lysosome-targeting peptides such as KFERQ (SEQ ID NO:112), QREFK (SEQ ID NO:113), and other pentapeptides having Q flanked on one side by four residues selected from K, R, D, E, F, I, V, and L Also included are targeting signals that direct insertion of the polypeptide into a membrane (e.g., a transmembrane sequence). Polypeptides including a membrane insertion sequence can be constructed either with or without a cytoplasmic tail.

An example of an ER-targeting sequence is the HLA-DRα leader sequence, Met Ala Ile Ser Gly Val Pro Val Leu Gly Phe Phe Ile Ile Ala Val Leu Met Ser Ala Gin Glu Ser Trp Ala (SEQ ID NO:63). The targeting sequence may include only a portion (e.g., at least ten amino acid residues) of this specified 25 residue sequence, provided that the portion is sufficient to cause targeting of the polypeptide to the ER.

Nuclear localization sequences include nudeoplasmin- and SV40-like nuclear targeting signals, as described in Chelsky et al., Mol. Cell Biol. 9:2487, 1989; Robbins, Cell 64:615, 1991, and Dingwall et al., TIBS 16:478, 1991. Some nuclear localization sequences include AVKRPAATICKAGOAKKK (SEQ ID NO:114), RPAATKKAGQAKKKKLD (SEQ ID NO:115), and AVKRPAATKKAGQAKKKLD (SEQ iD NO: 116).

In some cases it is desirable to modify the amino acid sequence of the targeting signal to facilitate cleavage by a signal peptidase or other proteolytic agent. Recognition sequences for signal peptidases are described In Von Heijne, Nucleic Acids Research 14:4683, 1986. The -3, -1 rules of von Heijne can be used to select a sequence that increases the probability of successful cleavage by signal peptidase when the targeting signal is present.

The nucleic acids encoding the polyepitope polypeptide described herein may optionally encode a methionine residue at the amino terminus of the polypeptide to facilitate translation.

If desired, a spacer amino acid or spacer sequence can be inserted between each pair of segments. Alanine spacers have been used successfully to separate multiple individual epitopes encoded in a single DNA construct (Toes et al., Proc. Nat. Acad. Sci. (USA) 94:14660-65, 1997).

Epitope-containing segments derived from a single protein can appear in the order (i.e., from amino terminus to carboxy terminus) in which they appear in the protein. Alternatively, segments from a given protein can be arranged in an order other than that in which they appear in the native protein, and can be grouped together or mixed with segments from one or more other proteins. The construct can encode a single polyepitope polypeptide or multiple polyepitope polypeptides, each under the control of a different promoter, e.g., dual promoter vectors. A dual promoter vector permits two shorter polyepitope polypeptides to replace the single longer version, with no loss in the numberof epitopes produced from a given vector. It also allows adding new epitopes without altering the sequence and perhaps the processing, of the first polyepitope polypeptide.

Nucleic acids encoding polyepitope polypeptides can be used in any vector that allows for expression in antigen-presenting cells (APC) of the patient. The vector is preferably a non-integrating viral vector or is a non-viral vector such as a plasmid or bacterial vector. An example of a suitable vector is the family of pcDNA mammalian expression vectors (Invitrogen), which permit direct and rapid cloning.of PCR products. The vector can be modified to include additional epitopes, e.g., MHC class I HLA-A1, -2, -3, -11, and -24 restricted epitopes from the HPV E6 and E7 proteins of the HPV 16 and 18 strains.

To determine whether the polypeptide is processed and the expected epitopes are presented by HLA, an *in vitro* T cell stimulation assay can be performed using autologous PBL or EBV-transformed cells infected with a recombinant vaccinia virus that contains the polyepitope polypeptide coding sequence. These target cells are generated by incubating PBL with the recombinant vaccinia at an m.o.i of 3-10 pfu/cell at 37°C for 2 h. After infection, cells are pelleted, washed and used as targets in the *in vitro* stimulation assay. The stimulated T cells from one or more individuals with the different HLA allotypes are incubated with the target cells, and the ability of the target cells to stimulate the T cells is measured, e.g., by γ-interferon expression or secretion.

Alternatively, epitope processing from the polyepitope polypeptide can be examined using proteasomes purified from human cells (Theobald et al., J. Exp. Med. 188:1017, 1998; Kisselev et al., J. Biol. Chem. 289:3363, 1999; and Nussbaum et al., Proc. Nat Acad. Sci. (USA) 95:12404, 1998).

In addition to the T cell assays, an assay that utilizes transgenic animals can be used to verify that the construct functions (e.g., epitopes are correctly processed and presented) when delivered *in vivo*. For measuring HLA-A2-restricted presentation, the polyepitope construct In a mammalian expression vector (e.g., a plasmid) is encapsulated in microspheres and introduced into HLA-A2 transgenic mice by a route such as intramuscular or subcutaneous injection. The construct may alternatively be administered without the microsphere delivery vehicle, e.g., in a recombinant vaccinia virus or as naked DNA. T cell responses are subsequently examined *in vitro* (Hedley et al., Nature Med. 4:365-68, 1998). Target cells are T2A2 cells (T2 cells transfected with DNA encoding HLA-A2) or EL4.A2 cells (EL4 cells transfected with DNA encoding HLA-A2) pulsed with the A2 epitope being tested and T2A2 cells into which has been introduced a nucleic acid of the invention. Parallel studies are performed using T2A2 cells pulsed with no peptide or with an irrelevant peptide. In this way, HLA-A2 epitopes that are processed and presented *in vivo* following administration of the nucleic acid of the invention are identified. A positive result suggests that processing of the polyepitope polypeptide is occurring as predicted.

The nucleic acid encoding the polyepitope polypeptide, as well as the polyepitope polypeptide itself, can be used in manufacture of a medicament for the prevention or treatment of a tumor or an infection with a pathogen (e.g., HPV), or conditions associated with such infection.

### Delivery of Epitopes and Nucleic Acids Encoding Immunogenic Epitopes

Various art-recognized delivery systems may be used to deliver polyepitope polypeptides, or nucleic acids encoding polyepitope polypeptides, into appropriate cells. An advantage of DNA delivery of antigenic MHC class I-restricted epitopes is that the epitopes are produced inside the target cell itself, where the interaction with a class I MHC molecule to which the immunogenic epitope binds is kinetically favored. This is in contrast to standard vaccine protocols which do not specifically direct antigenic epitopes to MHC molecules intracellularly so that the epitopes may bind with the MHC molecules prior to presentation of the MHC molecules on the cell surface.

The polyepitope polypeptides and nucleic acids encoding the polyepitope polypeptides can be delivered in a pharmaceutically acceptable carrier such as saline, or as colloidal suspensions, or as powders, with or without diluents. They can be "naked" or associated with delivery vehicles and delivered using delivery systems known In the art, such as lipids, liposomes, microspheres, microparticles or microcapsules, gold particles, ISCOMS, nanoparticles, polymers, condensing agents, polysaccharides, polyamino acids, dendrimers, saponins, QS21, adsorption enhancing materials, adjuvants, or fatty acids. Examples of suitable microparticles are presented below.

The polyepitope polypeptides, or nucleic acids encoding the polyepitope polypeptides, can be administered using standard methods, e.g., those described in Donnelly et al., J. Imm. Methods 176:145, 1994, and Vitiello et al., J. Clin. Invest 95:341, 1995, and can be delivered into subjects in any manner known In the art, e.g., orally intramuscularly, intravenously, intraarterially, intrathecally, intradermally, intraperitoneally, intranasally, intrapulmonarily, intraocularly, intravaginally, intrarectally or subcutaneously. They can be introduced into the gastrointestinal tract or the respiratory tract, e.g., by inhalation of a solution or powder containing the microparticles. Administration can be local (e.g., at the cervix, skin, or other site of HPV infection) or systemic.

It is expected that a dosage of approximately 0.1 to 100 µmoles of the polypeptide, or of about 1 to 2000 µg of DNA, would be administered per kg of body weight per dose. Where the patient is an adult human, vaccination regimens can include, e.g., intramuscular, intravenous, oral, or subcutaneous administrations of 10-1000 µg of a plasmid DNA when delivered in a microparticle, or of about 10-2500 µg, e.g., 100 to 2000, or 500 to 1000 µg, of naked plasmid DNA delivered Intramuscularly or intradermally, repeated 3-6 times. Of course, as is well known in the medical arts, dosage for any given patient depends upon many factors, including the patient's size, general health, sex, body surface area, age, the particular compound to be administered, time and route of administration, and other drugs being administered concurrently. Determination of optimal dosage is well within the abilities of a pharmacologist of ordinary skill.

Other standard delivery methods, e.g., biolistic transfer or *ex vivo* treatment, can also be used. In *ex vivo* treatment, antigen presenting cells (APCs) such as dendritic cells, peripheral blood mononuclear cells, or bone marrow cells can be obtained from a patient or an appropriate donor and activated *ex vivo* with the immunogenic compositions, and then implanted or reinfused into the patient.

The nucleic acids encoding the polyepitope polypeptides, or the polyepitope polypeptides themselves, can be administered alone or in combination with other therapies known in the art, e.g., chemotherapeutic regimens, radiation, and surgery, to treat tumors or infection, e.g., HPV infections or diseases associated with HPV infections In addition, the polypeptides and nucleic acids of the invention can be administered in combination with other treatments designed to enhance immune responses, e.g., by co-administration with adjuvants or cytokines (or nucleic acids encoding cytokines), as is well known in the art.

### Microsphere Delivery

In one preferred delivery method, nucleic acids encoding the polyepitope polypeptides, or the polyepitope polypeptides themselves, are delivered using microspheres. Microspheres, including those described in U.S. Patent No. 5,783,567, can be used as vehicles for delivering macromolecules such as DNA, RNA, or polypeptides into cells. The microspheres contain the macromolecules embedded in a polymeric matrix or enclosed in a hollow shell of polymer. Solid microspheres may also be formed for example as in WO 95/24929, herein incorporated by reference. The term microsphere, as used herein, includes microparticles and microcapsules. Microspheres act to maintain the integrity of the macromolecule, e.g., by maintaining the encapsulated DNA in a nondegraded state. Microspheres of an appropriate size or combination of sizes can also be used for pulsed delivery of the macromolecule, and for delivery at a specific site or to a specific target cell population.

The polymeric matrix can be a biodegradable polymer such as polylactide-co-glycolide(PLG), polylactide, polyglycolide, polyanhydride, polyorthoester, polycaprolactone, polyphosphazene, proteinaceous polymer, polypeptide, polyester, or a naturally occurring polymer such as starch, alginate, chitosan, and gelatin.

The microspheres can also include one or more stabilizer compounds (e.g., a carbohydrate, a cationic compound, a pluronic, e.g., Pluronic-F68^{™} (Slgma-Aldrich Co., St. Louis, MO), a lipid, or a DNA-condensing agent). A stabilizer compound is a compound that acts to protect the nucleic acid (e.g., to keep it supercoiled or protect it from degradation) at any time during the production of microspheres or after *in vivo* delivery. The stabilizer compound can remain associated with the DNA after a later release from the polymeric delivery system.

Examples of stabilizer compounds include tris(hydroxymethyl)aminomethane (TRIS), ethylenediamine-tetraacetic acid (EDTA), ora combination of TRIS and EDTA (TE). Otherstabilizercompounds include dextrose, sucrose, lactose, dextran, trehalose, cyclodextrin, dextran sulfate, cationic peptides, pluronics, e.g., Pluronic F-68^{™} (Sigma-Aldrich Co., St. Louis, MO), and lipids such as hexadecyltrimethylammonium bromide. Preparation of microspheres containing stabilizer agents is described in USSN 09/266,463.

The lipid can be a charged lipid such as a cationic lipid, an anionic lipid (such as PEG-DSPE, taurocholic acid or phosphatidyl inositol), or a zwitterionic lipid, or may have no charge. Examples of lipids include cetyltrimethylammonium and phospholipids, e.g., phosphatidylcholine. The microspheres may contain one or more than one type of lipid, e.g., those lipids present in lecithin lipid preparations, and may also include one or more stabilizer compounds as described above.

Microspheres can be used to maximize delivery of DNA molecules into a subject's phagocytotic cells. Alternatively, the biodegradable microspheres can be injected or implanted in a tissue, where they form a deposit. As the deposit breaks down, the nucleic acid is released gradually over time and taken up by neighboring cells (including APCs) as free DNA.

### Delivery Using Other Agents

The polyepitope polypeptides, or nucleic acids encoding them, can also be administered to subjects using other agents such as lipids, dendrimers, or liposomes, using techniques that are well known in the art. For example, liposomes carrying either immunogenic polypeptides or nucleic acids encoding immunogenic epitopes are known to elicit CTL responses *in vivo* (Reddy et al., J. Immunol. 148:1585, 1992; Collins et al., J. Immunol. 148:3336-3341, 1992; Fries et al., Proc. Natl. Acad. Sci. (USA) 89:358, 1992; Nabel et al., Proc. Natl. Acad. Sci. (USA) 89:5157, 1992).

The polypeptides and nucleic acids of the invention can also be administered by using Immune Stimulating Complexes (ISCOMS), which are negatively charged, cage-like structures 30-40nm in size formed spontaneously on mixing cholesterol and Quil A (saponin), or from saponin alone. The polypeptides and nucleic acids of the invention can be complexed with ISCOMs, then administered, or can be administered separately.

Protective immunity has been generated in a variety of experimental models of infection, including toxoplasmosis and Epstein-Barr virus-induced tumors, using ISCOMs as the delivery vehicle for antigens (Mowat et al., Immunology Today 12:383-385, 1991). Doses of antigen as low as 1 µg encapsulated In ISCOMs have been found to produce class I- mediated CTL responses, where either purified intact HIV-1-IIIB gp 160 envelope glycoprotein or influenza hemagglutinin is the antigen (Takahashi et al., Nature 344:873-875, 1990).

### Measuring Immune Responses

The ability of polyepitope polypeptides, or nucleic acids encoding them, to elicit an immune response in a host mammal can be assayed by using methods for measuring immune responses that are well known in the art For example, the generation of cytotoxic T cells can be demonstrated in a standard ⁵¹Cr release assay, by measuring intracellular cytokine expression or secretion, or by using MHC tetramers. Standard assays, such as ELISA or ELISPOT, can be used to measure cytokine profiles attributable to T cell activation. T cell proliferation can be measured using assays such as ³H-thymidine uptake and other assays known in the art. B cell responses can be measured using art recognized assays such as ELISA.

Other methodologies, e.g., digital imaging and cytologic, colposcopic and histological evaluations, can also be used to evaluate the effects of immunogenic epitopes, and of nucleic acids encoding the immunogenic epitopes, on pathogen-associated lesions, or on viral or other pathogen levels generally.

The following are examples of the practice of the invention. They are not to be construed as limiting the scope of the invention in any way.

### Example 1. Identification of HPV-derived MHC class 1-binding epitopes

HLA alleles are isolated from cell lines which express a single HLA-A allotype. 10-20 liters of each cell line (about 1-2 × 10¹¹ cells) are grown in complete RPMI-1640 media (10% FCS, HEPES, Pen/Strep, essential amino acids, glutamine) in rollerbottles, and the cells harvested by centrifugation (10-15gm wet weight cells/10 L culture). A membrane preparation is generated by lysing cells in 10 mM Tris, I mM DTT, 0.1 mM PMSF, for 30 min. Debris is pelleted, after which the cleared lysate is homogenized In lysis buffer and again pelleted. The pellet is homogenized in buffer containing 4% NP-40 and ultracentrifuged. The detergent-soluble material is used for HLA purification.

The solubilized membrane preparation is pumped through pre-clearing columns (chromatographic matrix and normal mouse serum-matrix) before the protein/ligand containing effluent is directed towards one, or a series of, specific immunoaffinity column(s). Immunoaffinity columns containing the pan anti-class I mAb W6/32 can be used to isolate class I molecules from the single allotype-expressing cell lines. Alternatively, allotype-specific mAb such as BB7.2 are used to extract a single HLA allotype (HLA-A2) from a cell lysate that expresses multiple allotypes. Coupling of mAb to high strength, large throughpore perfusion sorbents (coated and crosslinked with a hydrophilic stationary phase and covalently attached to Protein A) can be utilized to allow for fast flowrates (up to 20 ml/min). The immunoaffinity columns are then extensively washed and the protein/ligand complex is eluted from the immunoaffinity support using 50 mM carbonate/0.1% DOC/0.05% NaN₃ at pH 11.5.

Multi-modal high-performance liquid-chromatography (HPLC) separation of HLA molecules is achieved by coupling the chromatographic procedures in series with automated switching valves, which direct the protein/ligand-containing effluent to subsequent columns in the sequence. Each column effluent can be monitored at multiple UV wavelengths, pressure, and pH.

Purified HLA alleles are then used in epitope binding competition assays. Binding by a putative epitope is compared to binding by previously described HLA-binding epitopes. Examples of known epitopes and their respective allotypes include: HLA-A1, YLEPAIAKY (SEQ ID NO: 117); HLA-A2, FLPSDYFPSV (SEQ ID NO: 118); HLA-A3, KVF-PYALINK (SEQ ID NO:119); HLA-A11, AVDLYHFLK (SEQ ID NO:120); and HLA-A24, AYIDNYNKF (SEQ ID NO:121). General assay conditions are as follows: class I proteins are incubated in 0.05% NP-40/PBS with -5 nM of radiolabeled standard epitope and the test inhibitor epitope in presence of 1 µM human β2microglobulin, and a mixture of protease inhibitors (final concentration 1 mM PMSF, 1.3 mM 1,10 phenanthroline, 73 µM pepstatin, 8 mM EDTA and 200 nM N-α-p-tosyl-L-lysine chloromethyl ketone) at 23 °C for 48 h. The final concentration of each HLA allotype is determined experimentally as explained below. Concentrations of inhibitor peptides are titrated at concentration ranges from 1 nM to 100 µM.

Free and bound peptides are separated by HPLC size exclusion chromatography using a TSK 2000^{™} SEC column and 0.5% NP-40/PBS. The eluent is monitored for radioactivity and the fraction of peptide bound to HLA relative to the total amount of offered peptide is calculated from the ratio of peptide in the void volume to the total peptide recovered. The final concentration of HLA-A1, -A2, -A3, -A11, and -A24 to be used in subsequent binding assays must be determined experimentally based on the binding efficiency for the radiolabeled standard peptide. The necessary protein concentration for inhibition studies typically falls between 10-40 nM. Each allotype is tested for binding of the radiolabeled peptide by performing serial dilutions of the protein concentration from 1 nM to 1 µM of the HLA molecule at the beginning of the screening.

The binding affinity of potential peptide epitopes to human leukocyte antigen (HLA) receptors can be studied using a competitive binding assay (Sette et al., Molecular immunology31:813-822,1994). This assay requires (1) purified HLA receptors, (2) a synthetic peptide which binds to the HLA receptor of interest and contains a tyrosine amino acid in a location which can be labeled with radioactive iodine-125 without interrupting the peptide's ability to bind, (3) purified beta-2-microglobulin β₂m), and (4) synthetic peptide epitopes which are to be tested. The optimal experimental HLA receptorconcentrations are established by titrating the amount of HLA receptor in each binding assay required to achieve at least 10% binding of the total labeled peptide (fixed at 5nM) β₂m concentration is also fixed at 1 µM). With these three parameters (concentration of receptor, β₂m, and concentration of radiolabeled peptide) held constant, a titration of unlabeled test peptide is performed. Each binding reaction is incubated at room temperature for 30-80 h to allow for peptide exchange. The quantitation of the peptide exchange is determined by separating the bound fraction of radiolabeled peptide/receptor complex away from the excess free peptide and β₂m by size exclusion chromatography. Using an HPLC system fitted with a radioisotope detector, the percentage of radiolabeled peptide bound to the HLA receptor can be quantified. The ability of the unlabeled test peptide to Inhibit the binding of the labeled peptide is then plotted as a function of its concentration. The affinity of the test peptide is presented as the concentration of test peptide required to inhibit 50% of the total binding of the radiolabeled peptide (a so-called IC-50 value).

Several peptides derived from the E6 and E7 proteins of the human papilloma virus (HPV) strains 16 and 18 have been identified as high affinity, HLA-binding peptides using this type of assay. In these experiments, HLA receptors A1 and A11 were purified from human cells which had been transfected with HLA-A*0101 and HLA-A*1101 genes, respectively (Gorga et al., J. Biol. Chem. 262:16087-16094,1987). In the case of HLA receptors A3 and A24, recombinant HLA receptors (HLA-A*0301 and HLA-A*2402) were used which had been produced in E. coli and refolded (Garboczi et al., Proc. Natl. Acad. Sci. USA 89:3429-3433, 1992). Examples of experimental conditions and data collected are presented in Table 4.

**TABLE 4**

| Sequence (amino acid) | HLA Type | Incubation (hours) | IC-50 (nM) |
|---|---|---|---|
| ISEYRHYCY (SEQ ID NO:70) | A1 | 51 | 49 |
| IVYRDGNPY (SEQ ID NO:83) | A3 | 46 | 238 |
| HTMLCMCCK (SEQ ID NO: 122) | A11 | 68 | 67 |
| QLFLNTLSF (SEQ ID NO:123) | A24 | 68 | 47 |
| SLQDIEITCV (SEQ ID NO:130) | A2 | 50.5 | 50 |

If desired, the predictive binding algorithm described above can be used to prioritize putative epitopes according to dissociation times for each allele, with epitopes having the longest dissociation time tested first After 5-10 epitopes with reasonable affinities for each of the dominant H LA-A alleles (i.e., < 500nM) are identified, testing can be discontinued. If epitopes with less than 500nM binding affinity are not found, then the 5-10 best binders from each protein can be selected for continued analysis.

Peptides can also be identified, for example, according to the methods of Parker, et al. (J. Immunol. 149: 1896-1904, 1992; J. Immunol. 149: 2580-3587, 1992; J. Biol. Chem. 267:5451-5459, 1992) and Garboczi, et al. (PNAS 89:3429-3433, 1992).

### Example 2. Detection of MHC class I-restricted epitope presentation in vitro

Peripheral blood mononuclear cells (PBMC) from normal volunteers are purified by Ficoll-Paque^{™} (Pharmacia, Piscataway, NJ) density centrifugation from leukophoresis products screened as HIV, HBV, and HCV seronegative. Dendritic cells (DC) are generated in tissue culture from monocyte-enriched PBMC fractions as described (Tsai et al., Crit Rev. Immunol. 18:65-75, 1998; Wilson et al., J. Immunol. 162:3070-78, 1999). 10⁷ PBMC/ml in serum-free RPMI 1640 medium (Gibco-BRL) are plated in flasks and incubated 1.5-2.0 hr at 37°C. Non-adherent cells are removed by gentle washes, and the plastic-adherent monocytes containing DC precursors are cultured in complete medium in the presence of 50 ng/ml GM-CSF and 1000 U/ml IL-4 (both from R&D Systems, Minneapolis, MN) for 6-7 days. Complete medium consists of RPMI 1640 supplemented with 5% pooled human AB serum (C-6 Diagnostics, Mequon, WI), L-glutamine, penicillin/streptomycin, 2-ME (Gibco-BRL, Gaithersburg, MD) and HEPES (JRH Biosciences, Lenexa, KS) at the recommended concentrations.

Non-adherent cells are collected by centrifugation and prepared directly for use as APC or cryopreserved. Cells are determined to be DC by morphology and by expression of a CD3/CD16-negative, MHC class I^{hi}, MHC class II^{hi}, CD86-positive phenotype as assessed by cytofluorimetry.

At Day 0, 5x10⁵ non-adherent PBL are plated with 2.5x10⁴ irradiated, epitope-pulsed DC. The DC are in 48 well plates in 0.5 ml complete RPMI-1640 with 10% normal human AB serum supplemented with 10 ng/ml IL-7. The DC are incubated with the PBL at 37°C in a CO₂ incubator. 10 ng/ml IL-10 is added 24 h later. On day 7, autologous PBL are thawed and irradiated. 1x10⁶ cells per well are plated in a 48 well plate and allowed to adhere for 2 h. Epitopes are added, and cells are pulsed for 2 h. Cells are washed once, and responders transferred into wells with adherent stimulators. 10 ng/ml IL-10 is added 24 h later. 20 U IL-2/ml are added on day 9 and 100 U/ml IL-2 on day 11. Cells are restimulated on day 15 in the same fashion. On day 22, the cells in 1-2 wells of each individual culture are harvested and counted. Responders are those cells that have increased cell number 1.5-3 fold over day 16 input. The total culture yields generally should be at least 2x10⁶ cells.

Cells to be used as targets are harvested and adjusted to 6.67x10⁵ cells/ml in medium. Two mls of cells are pulsed with 50 ug/ml of each of the appropriate epitopes (e.g., Flu M1, Test #1, Test #2, Test #3. Test #4). Effectors are harvested and resuspended at 1x10⁶ cells/ml in medium. 100 µl is plated into each well of a round bottom 96-well plate. 150 µl of each respective target cell is aliquotted to the well containing the appropriate effector cells for a total volume of 250 µl/well. Plates are incubated for 24 h in an humidified 37°C CO₂ incubator.

ENDOGEN (Wobum, MA) human IFNγELISA kits are used to measure IFNγ secretion. The assay is performed according to manufacturer's directions, and supernatants are tested in duplicate. IFN-γ standards of 1000 pg/ml, 400 pg/ml, 160 pg/ml, 64 pg/ml, 25.6 pg/ml, and 0 pg/ml are tested in duplicate. The assay plate is read on a Molecular Devices Kinetic Microplate Reader and the results analyzed with Vmax Plate Reader/SOFTMAX™ software.

T2A2 cells are suitable targets when testing the HPV 16 and 18 E6 and E7 epitopes in the T cell assay (the HPV 2.4-C peptide (SEQ ID NO:61) is included as an example of an HLA-A2 binding HPV epitope to be tested in this way). However, when testing other allotypes (e.g., HLA-A1, -A3, -A11 and -A24), T2A2 cells are not appropriate APCs, and the FluM1 epitope is not an appropriate positive control epitope. Appropriate targets are autologous EBV-transformed cells or PBL. Appropriate control recall epitopes for these other allotypes include the following: HLA-A1, Flu NP 44-52; HLA-A3, Flu NP 265-273; HLA-At 1, EBNA3 603-611 and EBNA4 416-424; HLA-A24, EBV LMP 419-427. Suitable *in vitro* immunization controls include HLA-A1, MAGE- 61-69; HLA-A2, HBVpol 455-463; HLA-A3, P. *falciparum* LSA-94-102; HLA-A11, HIV gag 325-333; and HLA-A24, HIV gp41 584-591.

### Example 3. Generation of primary peptide-speclflc cytotoxic T-tymphocytes in vitro using peptide-pulsed autologous dendritic cells

The effectiveness of various HPV-derived peptides In generating CTL responses *in vitro* was determined by co-culturing peptide-pulsed dendritic cells (DC) with peripheral blood mononuclear cells (PBMC) from donors having defined HLA allotypes. The tested peptides included peptides having amino acids sequences corresponding to the following HPV protein amino acid sequences: HPV strain 16 E7 89-97 (SEQ ID NO:96); HPV strain 16 E6 92-101 (SEQ ID NO:77); HPV strain 18 E7 59-67 (SEQ ID NO: 122); HPV strain 18 E6 13-21 (SEQ ID NO: 124), and HPV strain 18 E6 97-106 (SEQ ID NO: 125).

Primary T cell responses were initiated using a modification of published protocols (Tsai et al., Crit. Rev. Immunol. 18:65-75, 1998; Wilson et al., J. Immunol. 162:3070-78, 1999). Peripheral blood mononuclear cells (PBMC) were purified from leukophoreis products from HLA-A-defined normal donors who screened as seronegative for human immunodeficiency virus and hepatitis B virus. Dendritic cells (DC) were pulsed for 2 h at 20°C with 20 µg/ml peptides in phosphate buffered saline supplemented with 3 µg/ml β2 microglobulin and were irradiated (3000 rad) before use. 2x10⁵ peptide-pulsed and washed DC were co-cultured with 2x10⁶ autologous non-adherent PBMC in 24-well culture plates in the presence of 10 ng/ml IL-7. One day later, cultures were supplemented with 10 ng/mi IL-10. On days 7 and 14, individual cultures were restimulated by transfer of non-adherent PBMC responder cells onto autologous irradiated monocytes pulsed with 20 µg/ml peptide as described above. 10 ng/ml IL-10 and 100 U/ml IL-2 were added 1 and 2 days, respectively, after each restimulation cycle.

The immune reactivity of the PBMC cultures was assessed by the ability to elicit specific IFN-γ secretion from day 21 peptide-sensitized cultures following their incubation with antigen presenting cells pulsed with either the immunizing peptide or an irrelevant antigenic peptide that specifically bound the same targeted HLA class I molecule. 10⁵ effector cells were incubated with 10⁵ autologous, irradiated peptide-pulsed PBMC for 24 h at 37°C in 200 µl complete medium. Supernatants from these cultures were measured for IFN-γ secretion using a commercial ELISA assay. The results are shown in Table 5. Data are presented as picograms of IFN-γ/ml. Specific reactivity by a PBMC culture was arbitrarily defined as at least 20 pg/ml per assay culture and a 1.5-fold or higher difference in IFN-γ secretion in response to the test peptide-pulsed versus the Irrelevant peptide-pulsed stimulator cells. FluM1 58-66 and the EBNA4 416-424 CTL peptide epitopes were used as A2 and A11 irrelevant controls, respectively.

**Table 5: In vitro induction of primary, peptide-speclflc cytotoxic T-lymphocytes using peptide-pulsed dendritic cells**

| | | IFN-γ release (pg/ml) | |
|---|---|---|---|
| | In vitro sensitization/ expansion with peptide | Stimulator cells pulsed with | |
| PBMC donor / HLA type | | irrelevant peptide | test peptide |
| P5 /HLA-A11, A31 | A11/HIV gag 325-333 | 634 | 1166 |
| P8 / HLA-A2, A28 | A2 / HBV pol 455-463 | 96 | 1824 |
| P5/ HLA-A11 , A3 1 | 16E7 89-97 | 41 | 1275 |
| | 16E6 92-101 | 38 | 259 |
| | 18E7 59-67 | 262 | 665 |
| P8 / HLA-A2, A28 | 18E6 13-21 | 61 | 1590 |
| | 18E6 97-106 | 77 | 1352 |
| Specific reactivity against HPV 16 and 18 E6 and E7 peptides by CTL induced with peptide-pulsed dendritic cells. | | | |

### Example 4. Generation of a nucleic acid encoding a polyepitope polypeptide

Once candidate epitopes are identified, a DNA fragment is generated that encodes the epitopes of interest. Sequences encoding the epitopes are generated by overlapping PCR orby ligating oligonucleotides. Regulatory elements including a promoter, a Kozak sequence, an initiating ATG, a stop codon, an intron and polyadenylation sequences are included in the construct.

A T7 polymerase promoter is also present at the 5' end of the coding sequence, and a sequence encoding a FLAG mAb (or other) determinant can be present either directly 5' of the first epitope coding sequence or 3' of the last epitope coding sequence. Constructs with and without a targeting signal (e.g., a leader peptide or endosomal/class II loading vesicle targeting sequence) are created. The -3, -1 rules of von Heijne (Nuc. Acids Res. 14:4683-4690, 1986) are followed to ensure the probability of successful cleavage by signal peptidase when the leader is present.

The PCR fragment is cloned into a TA vector (Invitrogen) which permits direct and rapid cloning of PCR products. The construct is sequenced and, if mistakes have been incorporated, the PCR is repeated under more stringent or otherwise optimized conditions. The PCR clone, or alternatively the DNA produced by ligation of oligonucleotides, is then cloned into a mammalian expression vector such as p3K or pcDNA (Invitrogen). In addition, the fragment is cloned into a vaccinia vector (such as pSC11) for generation of recombinant vaccinia virus (see below) and a bacterial expression vector (such as pGEX-5x (Pharmacia)) that permits expression of a GST fusion protein of the polyepitope polypeptide yeast or baculovirus expression vector. *In vitro* transcription/translation (Promega T7 TNT coupled transcription/trans- , lation kits) is performed according to the manufacturer's instructions. The translated protein, when analyzed by SDS-PAGE and autoradiography, has been shown to produce a polypeptide of the expected size.

### Example 5. Identification of processed epitopes from polyepitope proteins

The T cell stimulation assay described in Example 2 is used to determine which epitopes are processed from the polyepitope polypeptide encoded by the construct. Effectors generated from PBL stimulated *in vitro* with each of the epitopes encoded in the construct are tested for γIFN secretion when incubated with the appropriate targets. Targets can be autologous PBL cells or EBV-transformed cells infected with a recombinant vaccinia virus that encodes the polyepitope polypeptide (with and without the leader). Processing of the polyepitope polypeptide liberates the T cell epitopes, which are subsequently presented on the surface of the target cells.

Recombinant vaccinia was produced by standard procedures by inserting the polyepitope polypeptide-encoding construct into the Smal site of the pSC11 vector (Chakrabarti et al., Mol. Cell. Biol. 5:3403-09,1985) and then generating recombinant vaccinia by methods known in the art.

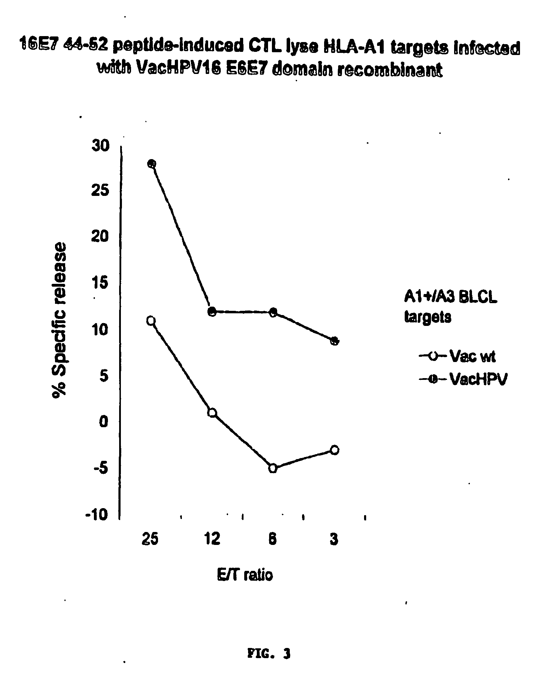

To determine if the encoded polyepitope polypeptide is processed and the expected epitopes are presented by HLA molecules, the *in vitro* stimulation assay may be performed as described in Example 2, with the exception that the targets are autologous EBV-transformed APCs Infected with the recombinant vaccinia virus that contains the polyepitope coding sequences. In the experiment illustrated in Fig. 3, EBV-transformed APCs from a donor were infected with recombinant vaccinia virus encoding the test peptide 16E7 44-52, at a m.o.i. of 5 pfu. Control cells were infected with wildtype vaccinia virus. The cells were incubated with the virus for 2.5 h in the presence of ⁵¹Cr. Following washing, the vaccinia-infected target cells were contacted for 5 h with autologous T cells previously activated with 16E7 44-52. ⁵¹Cr release into supernatant was taken as a measure of target cell lysis by the effector cells. As shown in Fig. 3, target cells infected with a vaccinia vector encoding the test peptide were more efficiently lysed by the effector cells than were the control target cells, suggesting that the peptide was expressed within the cells and effectively presented by the cells HLA molecules.

Targets can instead be autologous PBL Infected with the recombinant virus, or 7221 cells expressing a single HLA and which are transfected with the p3k or pcDNA construct (or any other mammalian expression vector) encoding the polyepitope polypeptide.

HLA-A2 transgenic animals may be used to verify that the construct functions to generate HLA-A2 epitopes when delivered *in vivo.* The polyepitope potypeptide-encoding construct In the mammalian expression vector is encapsulated in microparticles. They are injected into HLA-A2 transgenic mice, and T cell responses are subsequently examined as previously described (Hedley et al., Nature Med. 4:365-68,1998). Alternatively, the expression vectorcan be delivered as naked DNA. Target cells are HLA-2 expressing cells (e.g., T2A2 or EL4-A2 cells) pulsed with the HLA-A2 binding-epitope being tested, and similar cells infected with the potyepitope-encoding vaccinia vector. In this way, HLA-A2 epitopes that are processed and presented *in vivo* following administration of the construct are identified. Positive results Indicate that processing of the polyepitope polypeptide is occurring as predicted, though do not distinguish between epitopes presented by the mouse's endogenous murine MHC molecules and those presented by the transgene-derived HLA-A2 molecules. Immunization of the transgenic mice with a plasmid encoding a polyepitope polypeptide containing a a number of HPV-derived epitopes, some of which are known to bind HLA-A2 and at least one of which is known to bind to an endogenous murine MHC molecule, was shown to produce an immune response against two epitopes, including the one known to bind to the murine MHC molecule.

### Example 6: CTL responses generated in DNA-immunized HLA-A2 transgenic mice

CTL responses were measured in mice immunized with microsphere-encapsulated DNA encoding a polyepitope polypeptide. The DNA used for immunization was designated HPV Dra 16/18 (nucleotide sequence encoding the polypeptide of SEQ ID NO:160). Effector cells generated In the DNA Immunized mice were tested *in vitro* for their responsiveness In the presence of target cells infected with a vaccinia virus encoding a polyepitope polypeptide. Two polyepitope constructs were separately evaluated in target cells: (1) HPV Dra 16/18 (amino acid sequence of SEQ ID NO: 160); and (2) HPV 16/18(amino acid sequence of SEQ ID NO: 158).

Transgenic HLA-A *0201/H-2K^{b} line 6 mice (C57BL/8 x BIO.D2) originated from the breeding colony at the Research Institute of Scripps Clinic and were maintained under dean conventional conditions. HLA-A *0201/H-2K^{b} transgenic mice were injected intramuscularly In the hind limbs with 30 µg PEG-DSPE microsphere encapsulated p3KHPVDra 16/18 DNA. The mice were boosted on days 30 and 48 with 50 µg PEG-DSPE encapsulated p3KHPVDra 16/18 DNA.

On day 64, the mice were sacrificed and their spleens harvested. A single cell suspension was prepared, cells pooled, red blood cells lysed, and CD3+ enriched cell population obtained using an immune-affinity column (R&D Systems, Minneapolis, MN). Spleens cells were restimulated *in vitro* with three day old syngeneic Irradiated lipopolysaccharide (LPS)-stimulated B cell blasts (ratio 1:1) which had been preincubated for two hours at 37°C with 100 µM peptide In RPMI 1610 (JRH Biosciences, Lenexa, KS). Peptides used for *in vitro* expansion were: (1) HPV16E648-56 (peptide 124; EVYDFAFRD; SEQ ID NO:161): and (2) HPV16E786-93 (peptide 272; TLGIVCPI; SEQ ID NO:61). Peptides were dissolved In 100% DMSO to 20 mg/ml and stored at -20°C until use. In each well of a 6-well plate 10⁵ effectors were plated in RPMI 1610 supplemented with 10% fetal bovine serum (JRH Biosciences. Lenexa, KS), antibiotics (50 IU/ml penicillin and 50 µg/ml streptomycin), HEPES (JRH Biosciences, Lenexa, KS), 30 µM 2-ME (Gibco-BRL, Gaithersburg, MD) with final peptide concentration of 10 µM. On day 65,10 IU/ml miL-2 was added. Effectors were harvested on day 71 and assayed for peptide specific responses by measuring IFN-γ release in an ELISPOT assay, as described below.

The target cell line used in these experiments, an EL4 HLA-A2/H-2k^{b} cell line, was generated by transfecting EL4 cells (C57BL/6 thymoma, H-2^{b}) with a pSV2 plasmid containing the chimeric construct HLA-A2.1 (α₁ and α₂ domain) /H-2K^{b} (α₃ domain) and cotransfecting with the pSV2 neo plasmid containing the neomycin resistance gene.

Two recombinant vaccinia vectors (rVac), Vac HPV Dra 16/18 and Vac HPV 16/18, were generated by the insertion of either pSC11HPV Dra 16/18 or pSC1 1HPV 16/18 constructs into the thymidine kinase gene of wild type vaccinia virus (a TC-adapted Western Reserve strain; ATCC #VR1354). The resulting recombinant virus underwent three cycles of screening using dual selection with BrdU (Inactivation of thymidine kinase activity) and X-gal (pSG11 LacZ activity) in host cells with a TK- phenotype (143B, ATCC CRL-8303). Infected targets (EL4 HLA-A2/H-2K^{b} cells) were generated with 10 MOl rVac at 37°C for six hours.

A commercially prepared murine IFN-γ ELISPOT kit (R&D Systems, Minneapolis, MN) was utilized as per the manufacturer's suggested protocol. Each well of a 96-well hydrophobic polyvinylidene flouride (PVDF) membrane backed plate was pre-absorbed with anti-IFN-γ monoclonal antibody (mAb) and blocked with 10% RPMI for 20 minutes. Approximately 10⁴-10⁵ effectors were then mixed with 10⁵ targets (vaccinia Infected EL4 HLA-A2/H-2K^{b} cells) for 18-20 hours at 37°C in 5% CO₂, Next, each well was washed four times and incubated overnight at 4°C with a biotinylated non-competing anti-IFN-γ mAb. Wells were then washed three times, incubated for two hours at room temperature with streptavidin alkaline-phosphatase, washed again three times and developed with a 30 minute incubation with BCIP/NBT and washed extensively with distilled water. IFN-γ secreting cells (spots) were enumerated on an automated ELISPOT readersystem (Carl Zeiss Inc., Thornwood, NY) with KS ELISPOT Software 4.2 by Zellnet Consulting, Inc. (New York, NY).

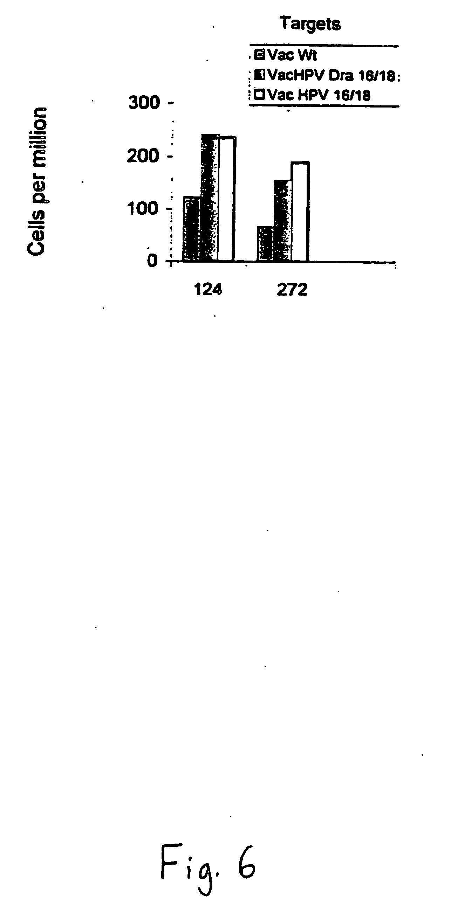

As shown if Figure 6, immunization of mice with DNA encoding an HPV polyepitope construct elicited peptide specific CTLs. Data are presented as the measurement of IFN-γ+ spots and represent the antigen specific response per million CD3+ cells.

### Example 7: CTL responses in fresh spleen cells from mice treated with DNA encoding a polyepitope polypeptide

Transgenic HLA-A *0201/H-2K^{b} mice (described in Example 6) were sequentially subjected to: (1) an injection of microsphere-encapsulated DNA encoding a polyepitope polypeptide; and (2) an infection with vaccinia virus encoding the polyepitope polypeptide. The IFN-γ ELISPOT assay described in Example 6 was used to detect and enumerate T cells specific for DNA-encoded CTL epitopes in fresh, unexpanded spleen cells.

The treatment regimen was as follows (see Table 6). Ten week old mice were injected with PEG/DSPE microspheres containing 100g of DNA. Twenty-six days after the microsphere injection, mice were infected intraperitoneally with 1x10⁷ plaque forming units of vaccinia virus encoding the same polyepitope polypeptide.

**Table 6: Vaccination Schedule and Immunization Regimen**

| Experimental Group | Number of Mice | Intramuscular DNA/ microsphere injection | Vaccinia Boost | intraperitoneal Virus dose |
|---|---|---|---|---|
| Untreated | 5 | none | none | none |
| Group 1 | 5 | pBKCMV | Vac wild type | 1x10⁷ PFU/0.1ml |
| Group2 | 5 | p3KDRaHPV1618 | VacDRα16-18 | 1x10⁷ PFU/0.1ml |

Nine days after the vaccinia boost, spleens were harvested, CD3+ T-cells were enriched (T-cell enrichment columns; R8D Systems, Minneapolis, MN), and peptide-specific IFN-γ release was detected using murine IFN-γ ELlSPOT (R&D Systems, Minneapolis, MN). For each antigenic treatment, five wells of 2.5x10⁵ T-enriched spleen cells were co-incubated with 2x10⁵ EL4-A2/K^{b} stimulator cells that were either untreated or pre-pulsed with defined class I peptide epitopes. As controls, spleen cells were also Incubated with stimulators either: (1) infected with 20mol of vaccinia wild type virus; or (2) treated with 25 ug Con A/ml. Plates were incubated at 37°C in 10% CO₂ for 48 hours and then developed for IFN-γ detection. HPV-specific IFN-γ responses were reported as the number of spot-forming cells (SFC)/1x10⁶ input T-enriched splenocytes. (the absolute numbers of SFCs are shown in Table 7). The background rate of IFN-γ secretion was defined as SFC/1x10⁶ input T-enriched splenocytes incubated with stimulator cells pulsed with irrelevant peptide (HLA-A2-restricted Plasmodium falciparum cp36 epitope; lot #322). HPV peptide-specific responses were considered positive if twice the background. The frequency of cp36-specific SFC/1x10⁶ cells was 0, 0, and 12 for Groups 0, 1, and 2, respectively.

**Table 7: IFN-γ Responses in Freshly Isolated Spleen Cells**

| Prime | Boost | HPV16 E648-56 | HPV16 E679-87 | HPV16 E749-57 | HPV16 E624-32 | HPV16 E77-15 |
|---|---|---|---|---|---|---|
| p3KDRa HPV1618 | VacHPVDrα 1618 | 124 | 37 | 73 | 24 | 34 |
| Vector | Vac wildtype | 14 | 10 | 2 | 5 | 16 |
| Untreated | none | 6 | 6 | 2 | 0 | 10 |

### Other Embodiments

While the invention has been described in conjunction with the detailed description thereof, the foregoing description is intended to illustrate and not limit the scope of the invention, which is defined by the scope of the appended claims.

## Claims

1. A nucleic acid encoding a hybrid polypeptide the sequence of which comprises a signal sequence and
(i) the following segments of human papilloma virus (HPV) strain 16 E6:
AMFQDPQERPRKLPQLCTEL (SEQ ID NO:64),
LLRREVYDFAFRDLCIVYRDGNPY (SEQ ID NO:65), and
KISEYRHYCYSLYGTTLEQQYNK (SEQ ID NO:66);
(ii) the following segments of HPV strain 16 E7:
TLHEYMLDLQPETTDLYSY (SEQ ID NO:67),
QAEPDRAHYNIVTF (SEQ ID NO:68), and
LLMOTLOIVCPICSQKP (SEQ ID NO:69);
(iii) the following segments of HPV strain 18 E6:
RRPYKLPDLCTELNTSLQDIEITCVYCKTVLELTEVFEFAFK (SEQ ID., NO:152), and
SVYGDTLEKLTNTGLYNLLIRCLRCQK (SEQ ID NO:153), and
(iv) the following segments of HPV strain 18 E7:
KATLQDIVLHLEPQNEIPV (SEQ ID NO:154),
HTMLCMCCKCEARI (SEQ ID NO:155), and
APQQLFLNTLSFVCPWC (SEQ ID NO:156),
provided that the hybrid polypeptide does not comprise a sequence Identical to the sequence of either full length, intact E6 or full length, intact E7 protein from HPV strain 16 or 18.

2. The nucleic acid of claim 1, wherein the signal sequence is the HLA-DRα leader sequence (SEQ ID NO:63).

3. The nucleic acid of claim 1, wherein the hybrid polypeptide comprises the amino acid sequence of SEQ ID NO:157.

4. The nucleic acid of claim 1, wherein the hybrid polypeptide comprises the amino acid sequence of SEQ ID NO:160.

5. A plasmid or viral vector comprising the nucleic acid of any one of claims 1 to 4.

6. A microsphere comprising a polymeric matrix or shell and the nucleic acid of any one of claims 1 to 4.

7. The microsphere of claim 6, wherein the polymeric matrix or shell consists essentially of a polymer of poly-co-glycolic acid (PLGA).

8. A therapeutic composition comprising the nucleic acid of any one of claims 1 to 4 and a pharmaceutically acceptable carrier.

9. The composition of claim 8, further including an adjuvant.

10. A liposome comprising the nucleic acid of any one of claims 1 to 4.

11. The hybrid polypeptide encoded by the nucleic acid of any one of claims 1 to 4.

12. The nucleic acid of any one of claims 1 to 4, the plasmid or viral vector of claim 5, the microsphere of claim 6 or 7, the therapeutic composition of claim 8 or 9, or the liposome of claim 10 for use as a medicament.

13. The nucleic acid of any one of claim 1 to 4, the plasmid or viral vector of claim 5, the microsphere of clalm 6 or 7. the therapeutic composition of claim 8 or 9, or the liposome of claim 10 for use in exciting an immune response in a mammal.

14. Use of the nucleic acid of any one of claims 1 to 4, the plasmid or viral vector of claim 5, the microsphere of claim 6 or 7, the therapeutic composition of claim 8 or 9, or the liposome of claim 10 for the manufacture of a medicament for eliciting an immune response in a mammal.

15. The nucleic acid, plasmid or viral vector, microsphere, therapeutic composition or liposome for use according to claim 13 or the use of claim 14, wherein the mammal is a human.

16. The nucleic acid of any one of claims 1 to 4, the plasmid or viral vector of claim 5, the microsphere of claim 6 or 7, the therapeutic composition of claim 8 or 9, or the liposome of claim 10 for use in treating or preventing exophytic condyloma, flat condyloma, cervical cancer, respiratory papilloma, conjunctival papilloma, genital-tract H PV infection, cervical dysplasia, high grade squamous intraepithelial lesions, or anal HPV infection.

17. Use of the nucleic acid of any one of claim 1 to 4, the plasmid or viral vector of claim 5 the microsphere of claim 6 or 7, the therapeutic composition of claim 8 or 9, or the liposome of claim 10 for the manufacture of a medicament for treating or preventing exophytic condyloma, flat condyloma, cervical cancer, respiratory papilloma, conjunctival papilloma, genital-tract HPV. infection, cervical dysplasia high grade aquarnous intraepithelial lesions, or anal HPV infection.

18. The nucleic acid, plasmid or viral vector, microsphere, therapeutic composition or liposome for use according to claim 15 or 16 or the use of claim 15 or 17, wherein the medicament is formulated for administration directly to mucosal tissue.

19. The nucleic acid, plasmid or viral vector, microsphere, therapeutic composition or liposome for use according to claim 18 or the use of claim 18, wherein the mucosal tissue is vaginal or anal tissue.

20. The nucleic acid, plasmid or viral vector, microsphere, therapeutic composition or liposome for use according to claim 15 or 16 or the use of claim 15 or 17, wherein the medicament is formulated for subcutaneous or intramuscular administration.

## Patentansprüche

1. Nukleinsäure, kodierend für ein Hybridpolypeptid, dessen Sequenz eine Signalsequenz umfasst sowie
(i) die folgenden Segmente des Human-Papilloma-Virus (HPV) Stamms 16 E6:
AMFQDPQERPRKLPQLCTEL (SEQ ID NO:64).
LLRREVYDFAFRDLCIVYRDGNPY (SEQ-ID NO:65), und
KISEYRHYCYSLYGTTLBQQYNK (SEQ ID NO:66);
(ii) die folgenden Segmente des HPV-Stamms 16 E7:
TLNEYMLDLQPBTTDLYSY (SEQ ID NO:67),
QAEPDRAHYNIVTF (SEQ ID NO:68), und
LLMGTLGIVCPICSQKP (SEQ ID NO:69);
(iii) die folgenden Segmente des HPV-Stamms 18 E6:
RRPYKLPDLCTELNTSLQDIEITCVYCKTVLELTEVFEFAFK (SEQ ID NO:152), und
SVYGDTLEKLTNTGLYNLLIRCLRCQK (SEQ ID NO:153), und
(iv) die folgenden Segmente des HPV-Stamms 18 E7:
KATLQDIVLHLEPQNEIPV (SEQ ID NO:154),
HTMLCMCCKCEARI (SEQ ID NO:155), und
AFQQLFLNTLSFVCPWC (SEQ ID NO:156),
mit der Maßgabe, dass das Hybridpolypeptid eine Sequenz nicht umfasst, welche identisch ist mit der Volllängen-Sequenz von intaktem E6- oder der Volllängen-Sequenz von intaktem E7-Protein von HPV-Stamm 16 oder 18.

2. Nukleinsäure nach Anspruch 1, worin die Signalsequenz eine HLA-DRα-Leadersequenz (SEQ ID NO:63) ist.

3. Nukleinsäure nach Anspruch 1, worin das Hybridpolypeptid die Aminosäuresequenz gemäß SEQ ID NO:157 umfasst.

4. Nukleinsäure nach Anspruch 1, worin das Hybridpolypeptid die Aminosäuresequenz SEQ ID NO:160 umfasst.

5. Plasmid oder Virusvektor, umfassend die Nukleinsäure nach einem der Ansprüche 1 bis 4.

6. Mikrosphäre, umfassend eine Polymermatrix oder -hülle und die Nukleinsäure nach einem der Ansprüche 1 bis 4.

7. Mikrosphäre nach Anspruch 6, worin die Polymermatrix oder -hülle im Wesentlichen aus einem Polymer aus Poly-co-glycolsäure (PLGA) besteht.

8. Therapeutische Zusammensetzung, umfassend die Nukleinsäure nach einem der Ansprüche 1 bis 4 und einen pharmazeutisch verträglichen Träger.

9. Zusammensetzung nach Anspruch 8, zusätzlich enthaltend ein Adjuvans.

10. Liposom, umfassend die Nukleinsäure nach einem der Ansprüche 1 bis 4.

11. Hybridpolypeptid, kodiert durch eine Nukleinsäure nach einem der Ansprüche 1 bis 4.

12. Nukleinsäure nach einem der Ansprüche 1 bis 4, Plasmid oder Virusvektor nach Anspruch 5, Mikrosphäre nach Anspruch 6 oder 7, therapeutische Zusammensetzung nach Anspruch 8 oder 9 oder Liposom nach Anspruch 10 zur Verwendung als Medikament.

13. Nukleinsäure nach einem der Ansprüche 1 bis 4, Plasmid oder Virusvektor nach Anspruch 5, Mikrosphäre nach Anspruch 6 oder 7, therapeutische Zusammensetzung nach Anspruch 8 oder 9 oder Liposom nach Anspruch 10 zur Verwendung bei der Bewirkung einer Immunantwort in einem Säuger.

14. Verwendung der Nukleinsäure nach einem der Ansprüche 1 bis 4, des Plasmids oder des Virusvektors nach Anspruch 5, der Mikrosphäre nach Anspruch 6 oder 7, der therapeutischen Zusammensetzung nach Anspruch 8 oder 9 oder des Liposoms nach Anspruch 10 zur Herstellung eines Medikaments zur Bewirkung einer Immunantwort in einem Säuger.

15. Nukleinsäure, Plasmid oder Virusvektor, Mikrosphäre, therapeutische Zusammensetzung oder Liposom zur Verwendung nach Anspruch 13 oder die Verwendung nach Anspruch 14, worin der Säuger ein Mensch ist.

16. Nukleinsäure nach einem der Ansprüche 1 bis 4, Plasmid oder Virusvektor nach Anspruch 5, Mikrosphäre nach Anspruch 6 oder 7, therapeutische Zusammensetzung nach Anspruch 8 oder 9 oder Liposom nach Anspruch 10 zur Verwendung bei der Behandlung oder Prävention von exophytischem Kondylom, flachem Kondylom, Zervikalkrebs, respiratorischem Papillom, konjunktivalem Papillom, Genitaltrakt-HPV-Infektion, zervikaler Dysplasie, hochgradigen intraepithelialen Läsionen oder analen HPV-Infektionen.

17. Verwendung der Nukleinsäure nach einem der Ansprüche 1 bis 4, Plasmid oder Virusvektor nach Anspruch 5, Mikrosphäre nach Anspruch 6 oder 7, therapeutische Zusammensetzung nach Anspruch 8 oder 9 oder Liposom nach Anspruch 10 zur Herstellung eines Medikaments zur Behandlung oder Prävention von exophytischem Kondylom, flachem Kondylom, Zervikalkrebs, respiratorischem Papillom, konjunktivalem Papillom, Genitaltrakt-HPV-Infektion, zervikaler Dysplasie, hochgradigen intraepithelialen Läsionen oder analen HPV-Infektionen.

18. Nukleinsäure, Plasmid oder Virusvektor, Mikrosphäre, therapeutische Zusammensetzung oder Liposom zur Verwendung nach Anspruch 15 oder 16 oder die Verwendung nach Anspruch 15 oder 17, worin das Medikament zur direkten Verabreichung an Mukosagewebe formuliert ist.

19. Nukleinsäure; Plasmid oder Virusvektor, Mikrosphäre, therapeutische Zusammensetzung oder Liposom zur Verwendung nach Anspruch 18 oder die Verwendung nach Anspruch 18, worin das Mukosagewebe vaginales oder anales Gewebe ist.

20. Nukleinsäure, Plasmid oder Virusvektor, Mikrosphäre, therapeutische Zusammensetzung oder Liposom zur Verwendung nach Anspruch 15 oder 16 oder die Verwendung nach Anspruch 15 oder 17, worin das Medikament zur subkutanen oder intramuskulären Verabreichung formuliert ist.

## Revendications

1. Acide nucléique codant pour un polypeptide hybride dont la séquence comprend une séquence signal et
(i) les segments suivants de la souche 16 E6 du papillomavirus humain (HPV) :
AMFQDPQERPRKLPQLCTEL (SEQ ID NO : 64),
LLRREVYDFAFRDLCIVYRDGNPY (SEQ ID NO : 65), et
KISEYRHYCYSLYGTTLEQQYNK (SEQ ID NO : 66) ;
(ii) les segments suivants de la souche 16 E7 du HPV :
TLHEYMLDLQPETTDLYSY (SEQ ID NO : 67),
QAEPDRAHYNIVTF (SEQ ID NO : 68), et
LLMGTLGIVCPICSQKP (SEQ ID NO : 69) ;
(iii) les segments suivants de la souche 18 E6 du HPV :
RRPYKLPDLCTELNTSLQDIEITCVYCKTVLELTEVFEFAFK (SEQ ID NO : 152), et
SVYGDTLEKLTNTGLYNLLIRCLRCQK (SEQ ID NO : 153), et
(iv) les segments suivants de la souche 18 E7 du HPV :
KATLQDIVLHLEPQNEIPV (SEQ ID NO : 154),
HTMLCMCCKCEARI (SEQ ID NO : 155), et
AFQQLFLNTLSFVCPWC (SEQ ID NO : 156),
à condition que le polypeptide hybride ne comprenne pas une séquence identique à la séquence de soit E6 intacte pleine longueur soit E7 intacte pleine longueur issue de la souche 16 ou 18 du HPV.

2. Acide nucléique selon la revendication 1, dans lequel la séquence signal est la séquence de tête de HLA-DRα (SEQ ID NO : 63).

3. Acide nucléique selon la revendication 1, dans lequel le polypeptide hybride comprend la séquence d'acides aminés de SEQ ID NO : 157.

4. Acide nucléique selon la revendication 1, dans lequel le polypeptide hybride comprend la séquence d'acides aminés de SEQ ID NO : 160.

5. Plasmide ou vecteur viral comprenant l'acide nucléique selon l'une quelconque des revendications 1 à 4.

6. Microsphère comprenant une enveloppe ou une matrice polymère et l'acide nucléique selon l'une quelconque des revendications 1 à 4.

7. Microsphère selon la revendication 6, dans laquelle l'enveloppe ou la matrice polymère est constituée essentiellement d'un polymère d'acide poly-co-glycolique (PLGA).

8. Composition thérapeutique comprenant l'acide nucléique selon l'une quelconque des revendications 1 à 4 et un véhicule pharmaceutiquement acceptable.

9. Composition selon la revendication 8, comprenant en outre un adjuvant.

10. Liposome comprenant l'acide nucléique selon l'une quelconque des revendications 1 à 4.

11. Polypeptide hybride codé par l'acide nucléique selon l'une quelconque des revendications 1 à 4.

12. Acide nucléique selon l'une quelconque des revendications 1 à 4, plasmide ou vecteur viral selon la revendication 5, microsphère selon la revendication 6 ou 7, composition thérapeutique selon la revendication 8 ou 9, ou liposome selon la revendication 10 pour une utilisation en tant que médicament.

13. Acide nucléique selon l'une quelconque des revendications 1 à 4, plasmide ou vecteur viral selon la revendication 5, microsphère selon la revendication 6 ou 7, composition thérapeutique selon la revendication 8 ou 9, ou liposome selon la revendication 10 pour une utilisation dans le déclenchement d'une réponse immunitaire chez un mammifère.

14. Utilisation de l'acide nucléique selon l'une quelconque des revendications 1 à 4, du plasmide ou du vecteur viral selon la revendication 5, de la microsphère selon la revendication 6 ou 7, de la composition thérapeutique selon la revendication 8 ou 9, ou du liposome selon la revendication 10 pour la fabrication d'un médicament destiné à déclencher une réponse immunitaire chez un mammifère.

15. Acide nucléique, plasmide ou vecteur viral, microsphère, composition thérapeutique ou liposome pour une utilisation selon la revendication 13 ou utilisation selon la revendication 14, dans laquelle le mammifère est un être humain.

16. Acide nucléique selon l'une quelconque des revendications 1 à 4, plasmide ou vecteur viral selon la revendication 5, microsphère selon la revendication 6 ou 7, composition thérapeutique selon la revendication 8 ou 9, ou liposome selon la revendication 10 pour une utilisation dans le traitement ou la prévention de condylomes exophytiques, de condylomes plats, d'un cancer du col de l'utérus, d'un papillome respiratoire, d'un papillome conjonctival, d'une infection par le HPV du système génital, d'une dysplasie du col de l'utérus, de lésions intra-épithéliales squameuses à un stade élevé ou d'une infection anale par le HPV.

17. Utilisation de l'acide nucléique selon l'une quelconque des revendications 1 à 4, du plasmide ou du vecteur viral selon la revendication 5 de la microsphère selon la revendication 6 ou 7, de la composition thérapeutique selon la revendication 8 ou 9, ou du liposome selon la revendication 10 pour la fabrication d'un médicament destiné au traitement ou à la prévention de condylomes exophytiques, de condylomes plats, d'un cancer du col de l'utérus, d'un papillome respiratoire, d'un papillome conjonctival, d'une infection par le HPV du système génital, d'une dysplasie du col de l'utérus, de lésions intra-épithéliales squameuses à un stade élevé ou d'une infection anale par le HPV.

18. Acide nucléique, plasmide ou vecteur viral, microsphère, composition thérapeutique ou liposome en vue d'une utilisation selon la revendication 15 ou 16 ou l'utilisation selon la revendication 15 ou 17, dans laquelle le médicament est formulé pour une administration directement dans du tissu mucosal.

19. Acide nucléique, plasmide ou vecteur viral, microsphère, composition thérapeutique ou liposome pour une utilisation selon la revendication 18 ou l'utilisation selon la revendication 18, dans laquelle le tissu mucosal est du tissu vaginal ou anal.

20. Acide nucléique, plasmide ou vecteur viral, microsphère, composition thérapeutique ou liposome pour une utilisation selon la revendication 15 ou 16 ou l'utilisation selon la revendication 15 ou 17, dans laquelle le médicament est formulé pour une administration sous-cutanée ou intramusculaire.
